(19) Europäisches Patentamt / European Patent Office / Office européen des brevets

(11) **EP 4 401 750 B1**

(12) **EUROPEAN PATENT SPECIFICATION**

(45) Date of publication and mention
of the grant of the patent:
**28.01.2026 Bulletin 2026/05**

(21) Application number: **22789185.0**

(22) Date of filing: **14.09.2022**

(51) International Patent Classification (IPC):
*A61K 38/01* *(2006.01)*     *A61K 38/20* *(2006.01)*
*A61K 39/00* *(2006.01)*     *A61P 27/06* *(2006.01)*

(52) Cooperative Patent Classification (CPC):
**A61P 27/06; A61K 38/20; A61K 45/06;
C07K 16/22;** A61K 2039/505; C07K 2317/24

(86) International application number:
**PCT/EP2022/075505**

(87) International publication number:
**WO 2023/041569 (23.03.2023 Gazette 2023/12)**

(54) **A COMPOSITION COMPRISING IL-36 AND OPTIONALLY IL-18 FOR USE IN TREATING OCULAR DISORDERS**

ZUSAMMENSETZUNG ENTHÄLTEND IL-36 UND OPTIONAL IL-18 ZUR VERWENDUNG IN DER BEHANDLUNG VON AUGENERKRANKUNGEN

COMPOSITION COMPRENANT IL-36 ET ÉVENTUELLEMENT 'IL-18 POUR L'UTILISATION DANS LE TRAITEMENT DE TROUBLES OCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2021 EP 21196717**

(43) Date of publication of application:
**24.07.2024 Bulletin 2024/30**

(73) Proprietor: **The Provost, Fellows, Foundation
Scholars, & the
other members of Board, of the College of the
Holy
& Undiv. Trinity of Queen Elizabeth near Dublin
Dublin 2 (IE)**

(72) Inventors:
• **DOYLE, Sarah**
  **Dublin 15 (IE)**
• **ADAMSON, Peter**
  **Broadstairs Kent CT10 3QY (GB)**
• **CAMPBELL, Matthew**
  **Dublin 15 (IE)**

(74) Representative: **FRKelly
Waterways House
Grand Canal Quay
Dublin D02 PD39 (IE)**

(56) References cited:
**WO-A1-2014/074823**     **WO-A1-2018/175752**

• **FAHEY ERIN: "Investigating the role of IL-36
cytokines in mediating angiogenesis and
vascular permeability", 1 March 2021
(2021-03-01), XP055905687, Retrieved from the
Internet <URL:http://www.tara.tcd.ie/bitstream/
handle/2262/97194/Erin_Fahey_Thesis.pdf?
sequence=1&isAllowed=y> [retrieved on
20220328]**
• **WOOFF YVETTE ET AL: "IL-1 Family Members
Mediate Cell Death, Inflammation and
Angiogenesis in Retinal Degenerative Diseases",
vol. 10, 16 July 2019 (2019-07-16), Lausanne, CH,
XP055899771, ISSN: 1664-3224, Retrieved from
the Internet <URL:https://www.ncbi.nlm.nih.gov/
pmc/ivip/1664-3224> [retrieved on 20220401],
DOI: 10.3389/fimmu.2019.01618**

**(Cont. next page)**

• MURRIETA-COXCA JOSÉ ET AL: "IL-36 Cytokines: Regulators of Inflammatory Responses and Their Emerging Role in Immunology of Reproduction", vol. 20, no. 7, 3 April 2019 (2019-04-03), Basel, CH, pages 1649, XP055899640, ISSN: 1661-6596, Retrieved from the Internet <URL:https://www.ncbi.nlm.nih.gov/pmc/ivip/1422-0067/20/7/1649> [retrieved on 20220401], DOI: 10.3390/ijms20071649

• PATRICK T WALSH ET AL: "The emergence of the IL-36 cytokine family as novel targets for inflammatory diseases", ANNALS OF THE NEW YORK ACADEMY OF SCIENCES, NEW YORK ACADEMY OF SCIENCES, US, vol. 1417, no. 1, 26 October 2016 (2016-10-26), pages 23 - 34, XP071410231, ISSN: 0077-8923, DOI: 10.1111/NYAS.13280

**Description**

**Field of the Invention**

The present invention relates to a composition comprising IL-36 and optionally IL-18 for use in treating ocular disorders such as age-related macular degeneration (AMD), macular oedema, retinopathy, diabetic retinopathy, and glaucoma.

**Background to the Invention**

Increased vascular and microvascular permeability is a key driver of organ injury in diseases, and strategies that regulate endothelial cell integrity are promising therapeutic targets. Blood vessels in the central nervous system possess unique barrier properties that prevent infiltration of unwanted substances and allow for precise delivery of ions, molecules, and immune cells into neural tissues.

Barrier breakdown is implicated in neurological and retinal conditions because the nascent vessels that contribute to disease often have a barrier incapable of maintaining the integrity of the vasculature, allowing for pathological leakage. Although many agents and disease processes induce vascular permeability, only a handful of factors have been discovered specifically to enhance barrier function and make vessels resistant to leakage.

In the eye, endothelial cells lining the microvasculature of the retina and choroid maintain the barrier between blood and tissue, functioning to maintain homeostasis. Loss of endothelial barrier-integrity and 'de novo' blood-vessel growth, termed 'angiogenesis' is almost exclusively associated with pathology in an adult. Disorders associated with vascular-leakage and angiogenesis are diverse and include the blinding conditions age-related macular degeneration (AMD), diabetic retinopathy and retinopathy of prematurity. AMD is the leading cause of central vision loss in the over 50's worldwide. In Ireland alone, out of a population of 4.6 million, over 1 million are over the age of 50, and over 70,000 have AMD, already the most common cause of registered blindness in Ireland. Globally it is estimated that between 30 and 50 million people have AMD. There are two end stages of disease, the most severe of which is neovascular or 'wet' AMD. In this form of the condition, unwanted blood vessels sprout from the underlying choroidal vasculature disrupting the integrity of the retina and causing instant vision loss. Approximately 10-17% of patients develop wet AMD.

Wet AMD can be treated acutely with regular monthly intraocular injections of antibodies or fusion proteins directed against vascular endothelial growth factor (VEGF). These treatments have revolutionized AMD therapy, stabilizing the areas of choroidal neovascularization (CNV) and delaying further neovascularization. However, there is no end stage to treatment, with patients requiring regular intraocular injections to sustain efficacy. Furthermore, a significant proportion of patients, estimated at approximately 30%, become refractory to treatment after 12 months, with a further proportion of patients never responding to treatment. Evidence also suggests that long-term use of anti-VEGF agents is damaging to the retina.

Other mechanistic approaches which do not target VEGF, but are used in combination with anti-VEGF therapeutics, such as anti-PDGF aptamers (such as Fovista® from Ophthotech) have proved successful in clinical studies to date, but recently failed Phase III studies for neovascular AMD. The use of anti-PDGF aptamers, dosed between anti-VEGF doses, was suggested to sensitize the infiltrating choroidal neovessels to the anti-VEGF therapeutic resulting in enhanced efficacy and demonstrating effective interaction between two independent mechanisms. However, these trials have proved not to be fruitful and better combination therapies are desperately needed.

Anti-VEGF therapies are also utilised for proliferative diabetic retinopathy and macular oedema.

However, they are less successful at stabilising vascular leakage and proliferation in these disorders.

Current "state of the art" anti-VEGF therapies (Lucentis® from Roche/Genentech and Eylea ® from Regeneron) are akin to sponges mopping up VEGF in the local microenvironment of the eye.

Furthermore, evidence also suggests that long-term use of anti-VEGF agents is damaging to the retina due to VEGFs role as a neuronal survival factor and is underscored by the observation that in patients receiving long-term anti-VEGF therapy the neural retina can begin to degenerate resulting in further irreversible vision loss. Tailoring therapies to attenuate specific signal transduction cascades, reducing the side effects of anti-VEGF agents holds significant therapeutic promise. VEGF Receptor-2 (VEGFR2) signalling appears to be mainly involved in promoting vascular permeability and angiogenesis, whereas VEGFR1 promotes cell survival, therefore designing therapies that block VEGFR2 signalling specifically would have the most impact.

Wooff et al. 2019 Jul 16;10:1618. doi: 10.3389/fimmu.2019.01618. PMID: 31379825; PMCID: PMC6646526 describes the contribution of IL-1 family members, in particular IL-1β and IL-18, to cell death, inflammation and angiogenesis in retinal regenerative diseases.

Thus there remains a clinical need for effective therapies to target ocular disorders.

## Summary of the Invention

According to the present invention, there is provided a composition comprising IL-36 for use in the treatment or prophylaxis of ocular disorders.

According to a further aspect of the present invention, there is provided a composition comprising IL-36 and IL-18 for use in the treatment or prophylaxis of ocular disorders.

Optionally, the use comprises administering the composition to a subject in need thereof. Further optionally, the use comprises administering the composition comprising IL-36 to a subject in need thereof. Still further optionally, the use comprises administering the composition comprising IL-36 and IL-18 to a subject in need thereof.

Optionally, the use further comprises administering an anti-VEGF agent to a subject in need thereof. Further optionally, the use comprises administering an anti-VEGF agent and the composition comprising IL-36 to a subject in need thereof. Still further optionally, the use comprises administering an anti-VEGF agent and the composition comprising IL-36 and IL-18 to a subject in need thereof.

Optionally, the anti-VEGF agent and the composition are administered simultaneously. Further optionally, the anti-VEGF agent and the composition comprising IL-36 are administered simultaneously. Still further optionally, the anti-VEGF agent, the composition comprising IL-36, and the composition comprising IL-18 are administered simultaneously.

Optionally, the anti-VEGF agent and the composition are administered sequentially. Further optionally, the anti-VEGF agent and the composition comprising IL-36 are administered sequentially. Still further optionally, the anti-VEGF agent, the composition comprising IL-36 and the composition comprising IL-18 are administered sequentially.

Optionally, the anti-VEGF agent is administered prior to the composition. Further optionally, the anti-VEGF agent is administered prior to the composition comprising IL-36. Still further optionally, the anti-VEGF agent is administered prior to the composition comprising IL-36 and the composition comprising IL-18.

Optionally, the composition is administered prior to the anti-VEGF agent. Further optionally, the composition comprising IL-36 is administered prior to the anti-VEGF agent. Still further optionally, the composition comprising IL-36 and the composition comprising IL-18 are administered prior to the anti-VEGF agent.

Optionally, the use further comprises administering an anti-IL-1 agent to a subject in need thereof.

Further optionally, the use comprises administering an anti-IL-1 agent and the composition comprising IL-36 to a subject in need thereof. Still further optionally, the use comprises administering an anti-IL-1 agent and the composition comprising IL-36 and IL-18 to a subject in need thereof. Still further optionally, the use comprises administering an anti-IL-1 agent, the anti-VEGF agent, and the composition comprising IL-36 to a subject in need thereof. Still further optionally, the use comprises administering an anti-IL-1 agent, the anti-VEGF agent, and the composition comprising IL-36 and IL-18 to a subject in need thereof.

Optionally, the anti-VEGF agent and the anti-IL-1 agent are administered simultaneously. Optionally, the anti-VEGF agent and the anti-IL-1 agent are administered sequentially. Optionally, the anti-VEGF agent is administered prior to the anti-IL-1 agent. Optionally, the anti-IL-1 agent is administered prior to the anti-VEGF agent.

Optionally, the IL-1 is selected from IL-1α and IL-1β.

Optionally, the anti-IL-1 agent is selected from IL-1Ra or fragment thereof, an anti-IL-1 antibody or fragment thereof, an anti-IL-1 polyclonal antibody or fragment thereof, an anti-IL-1 monoclonal antibody or fragment thereof, an anti-IL-1 inhibitor, a small molecule anti-IL-1 inhibitor, fusion proteins comprising IL-1 or fragment thereof, recombinant fusion proteins comprising IL-1 or fragment thereof, fusion proteins comprising IL-1 receptor accessory protein (IL-1RAcP) or fragment thereof, recombinant fusion proteins comprising IL-1 receptor accessory protein (IL-1RAcP) or fragment thereof, and combinations each thereof.

Optionally, the anti-IL-1 agent is selected from anakinra, canakinumab, rilonacept, and combinations each thereof.

Optionally, the use is use in compensatory proliferation, vessel maturation, mesenchymal activation, mesenchymal contraction, and/or in ocular disorders. Further optionally, the use is use in wound healing in ocular disorders.

Optionally, the use is use in microvascular remodelling in ocular disorders.

Optionally, the use is use in endothelial cell migration in ocular disorders.

Optionally, the use is use in increasing monolayer integrity in ocular disorders.

Optionally, the use is use in reducing vascular permeability. Further optionally, the use is use in reducing vascular permeability in ocular disorders.

Optionally, the use is use in increasing expression of tight junction and/or cadherin junction components in ocular disorders. Still further optionally, the use is use in increasing expression of tight junction and/or cadherin junction components selected from VE-cadherin, ZO-1, tricellulin, occluding, claudin-5, and combinations each thereof in ocular disorders.

Optionally, the use is use in increasing barrier function in the micro-vasculature of the CNS in ocular disorders.

Optionally, the use is use in stabilising pathological vascular permeability in ocular disorders.

Optionally, the use is use in reducing microvascular leakage in ocular disorders.

Optionally, the use comprises administration of a cell expressing IL-36 and optionally IL-18, and/or a receptor each thereof. Further optionally, the use comprises administration of a stem cell expressing IL-36 and optionally IL-18, and/or a receptor each thereof. Still further optionally, the use comprises administration of a mesenchymal stem cell expressing IL-36 and optionally IL-18, and/or a receptor each thereof.

Optionally, the use comprises administration of a cell adapted to express IL-36 and optionally IL-18, and/or a receptor each thereof. Further optionally, the use comprises administration of a stem cell adapted to express IL-36 and optionally IL-18, and/or a receptor each thereof. Still further optionally, the use comprises administration of a mesenchymal stem cell adapted to express IL-36 and optionally IL-18, and/or a receptor each thereof.

Optionally, the use comprises administration of a cell adapted to over-express IL-36 and optionally IL-18, and/or a receptor each thereof. Further optionally, the use comprises administration of a stem cell adapted to over-express IL-36 and optionally IL-18, and/or a receptor each thereof. Still further optionally, the use comprises administration of a mesenchymal stem cell adapted to over-express IL-36 and optionally IL-18, and/or a receptor each thereof.

Optionally, the ocular disorders are disorders associated with or caused by oedema and/or neovascularisation.

Optionally, the ocular disorders are selected from any one or more of age-related macular degeneration (AMD), nascent geographic atrophy, incomplete retinal pigment epithelium and outer retinal atrophy (iRORA), wet AMD, geographic atrophy, dry AMD, macular oedema, retinopathy, diabetic retinopathy and glaucoma.

Optionally, the anti-VEGF agent is selected from an anti-VEGF antibody or fragment thereof, an anti-VEGF polyclonal antibody or fragment thereof, an anti-VEGF monoclonal antibody or fragment thereof, a VEGF inhibitor, a small molecule VEGF inhibitor, a tyrosine kinase inhibitor, a small molecule tyrosine kinase inhibitor, fusion proteins comprising VEGF or fragment thereof, recombinant fusion proteins comprising VEGF or fragment thereof, and combinations each thereof.

Optionally, the anti-VEGF agent is selected from aflibercept, axitinib, bevacizumab, cabozantinib, lapatinib, lenvatinib, pazopanib, ponatinib, ramucirumab, ranibizumab, regorafenib, sorafenib, sunitinib, vandetanib, and combinations each thereof.

Preferably, the anti-VEGF agent is selected from aflibercept, bevacizumab, ranibizumab, and combinations each thereof.

Optionally, the use comprises administration by a route selected from topical, parenteral, intra-arterial, intravenous, intraocular, intravitreal, and combinations each thereof.

Preferably, the use comprises administration by a route selected from intraocular, intravitreal, and combinations each thereof.

Optionally, the use comprises administration of the composition by a route selected from topical, parenteral, intra-arterial, intravenous, intraocular, intravitreal, and combinations each thereof. Further optionally, the useermethod comprises administration of the composition comprising IL-36 by a route selected from topical, parenteral, intra-arterial, intravenous, intraocular, intravitreal, and combinations each thereof. Still further optionally, the useermethed comprises administration of the composition comprising IL-18 by a route selected from topical, parenteral, intra-arterial, intravenous, intraocular, intravitreal, and combinations each thereof.

Preferably, the use comprises administration of the composition by a route selected from intraocular, intravitreal, and combinations each thereof. Further preferably, the use comprises administration of the composition comprising IL-36 by a route selected from intraocular, intravitreal, and combinations each thereof. Still further preferably or additionally, the use comprises administration of the composition comprising IL-18 by a route selected from intraocular, intravitreal, and combinations each thereof.

Optionally, the use comprises administration of the anti-VEGF agent by a route selected from topical, parenteral, intra-arterial, intravenous, intraocular, intravitreal, and combinations each thereof.

Preferably, the use comprises administration of the anti-VEGF agent by a route selected from intraocular, intravitreal, and combinations each thereof.

Optionally, the use comprises administration at a dose of at least 0.3 ng/ml. Further optionally, the use comprises administration at a dose of at least 3 ng/ml. Still further optionally, the use comprises administration at a dose of at least 30 ng/ml.

Optionally, the use comprises administration at a unit dose of at least 0.3 ng. Further optionally, the use comprises administration at a unit dose of at least 3 ng. Still further optionally, the use comprises administration at a unit dose of at least 30 ng.

Optionally, the use comprises administration at a unit dose of at least 0.3 ng/eye. Further optionally, the use comprises administration at a unit dose of at least 3 ng/eye. Still further optionally, the use or method comprises administration at a unit dose of at least 30 ng/eye.

Optionally, the use comprises administration of the composition at a dose of at least 0.3 ng/ml. Further optionally, the use comprises administration of the composition at a dose of at least 3 ng/ml. Still further optionally, the use comprises administration of the composition at a dose of at least 30 ng/ml.

Optionally, the use comprises administration of the composition at a unit dose of at least 0.3 ng. Further optionally, the use comprises administration of the composition at a unit dose of at least 3 ng. Still further optionally, the use comprises administration of the composition at a unit dose of at least 30 ng.

Optionally, the use comprises administration of the composition at a unit dose of at least 0.3 ng/eye. Further optionally, the use comprises administration of the composition at a unit dose of at least 3 ng/eye. Still further optionally, the use comprises administration of the composition at a unit dose of at least 30 ng/eye.

Optionally, the use comprises administration of the composition comprising IL-36 at a dose of at least 0.3 ng/ml. Further optionally, the use comprises administration of the composition comprising IL-36 at a dose of at least 3 ng/ml. Still further optionally, the use comprises administration of the composition comprising IL-36 at a dose of at least 30 ng/ml.

Optionally, the use comprises administration of the composition comprising IL-36 at a unit dose of at least 0.3 ng. Further optionally, the use comprises administration of the composition comprising IL-36 at a unit dose of at least 3 ng. Still further optionally, the use comprises administration of the composition comprising IL-36 at a unit dose of at least 30 ng.

Optionally, the use comprises administration of the composition comprising IL-36 at a unit dose of at least 0.3 ng/eye. Further optionally, the use comprises administration of the composition comprising IL-36 at a unit dose of at least 3 ng/eye. Still further optionally, the use comprises administration of the composition comprising IL-36 at a unit dose of at least 30 ng/eye.

Optionally, the use comprises administration of the composition comprising IL-18 at a dose of at least 0.3 ng/ml. Further optionally, the use comprises administration of the composition comprising IL-18 at a dose of at least 3 ng/ml. Still further optionally, the use comprises administration of the composition comprising IL-18 at a dose of at least 30 ng/ml.

Optionally, the use comprises administration of the composition comprising IL-18 at a unit dose of at least 0.3 ng. Further optionally, the use comprises administration of the composition comprising IL-18 at a unit dose of at least 3 ng. Still further optionally, the use comprises administration of the composition comprising IL-18 at a unit dose of at least 30 ng.

Optionally, the use comprises administration of the composition comprising IL-18 at a unit dose of at least 0.3 ng/eye. Further optionally, the use comprises administration of the composition comprising IL-18 at a unit dose of at least 3 ng/eye. Still further optionally, the use comprises administration of the composition comprising IL-18 at a unit dose of at least 30 ng/eye.

Optionally, the IL-36 is selected from IL36$\alpha$, IL36$\beta$ or IL36y.

Preferably, the IL-36 is selected from IL36$\beta$.

## Brief Description of the Drawings

The invention will now be described with reference to the accompanying drawings in which:

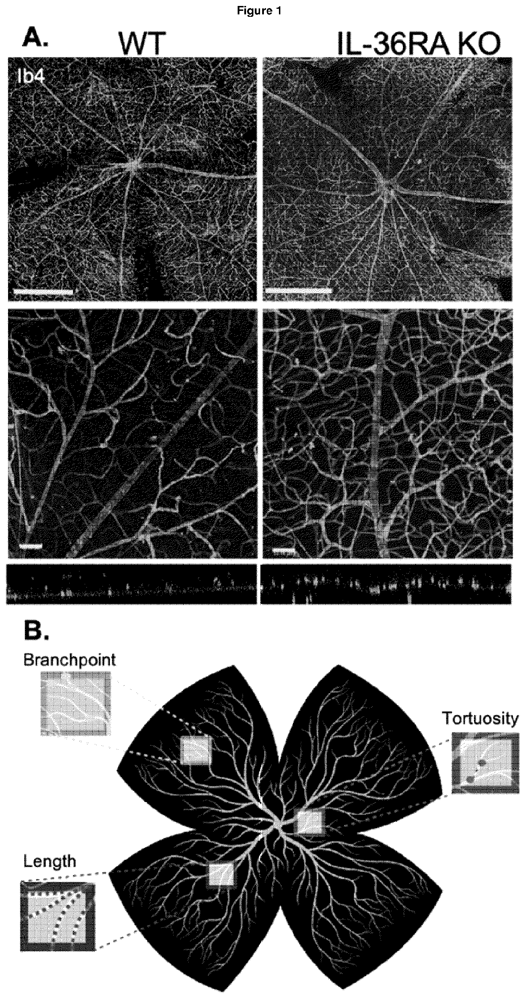

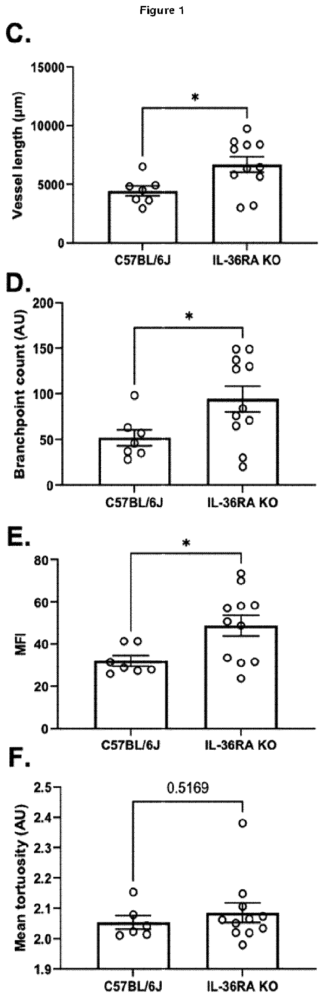

Figure 1 illustrates overt IL-36 signalling leads to increased retinal vascular density, wherein (A) is isolectin staining in Z-stacks of retinal flatmounts highlighting the vasculature of 12 month old C57BL/6J wild type mice compared to IL-36rn-/- mice, representative of N=2 mice for wild-type and N=4 mice for IL-36rn-/-; (B) is schematic illustration of quantifiable measures in retinal flatmount using REAVER analysis, indicating vessel length, branch points and retinal tortuosity i.e. the distance required to go from point to point; (C) is vessel length; (D) is branch point number as measured using the REAVER program on representative flattened Z-stacks taken at 20x magnification of isolectin mstained retinal flatmounts from wild-type (WT) and IL-36rn-/- (IL-36RA KO); (E) is MFI of isolectin stained retinal flatmount flattened Z-stack images taken at 20x magnification from WT or IL-36RA KO mice; (F) is retinal tortuosity as measured using the REAVER program on representative flattened Z-stacks taken at 20x magnification of isolectin stained retinal flatmounts from WT and IL-36RA KO mice, wherein all statistical analysis was performed using t-tests, were p<0.05 = *;

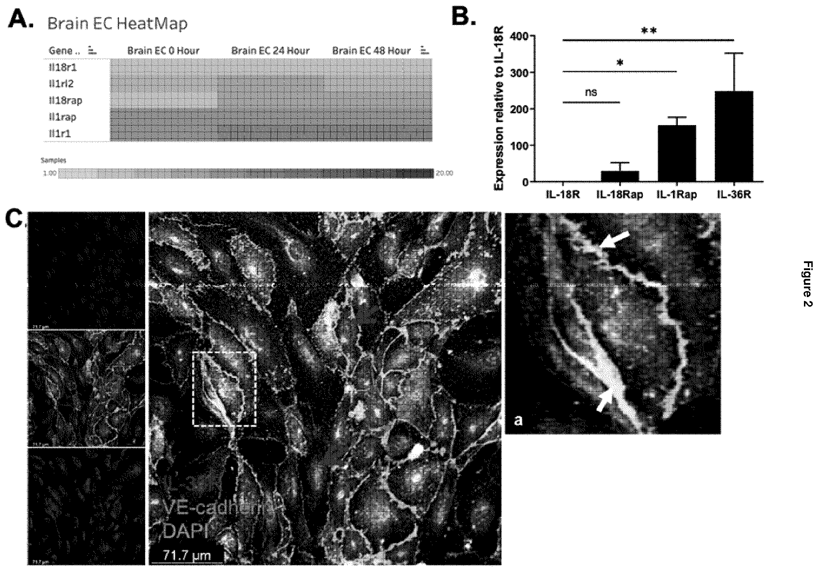

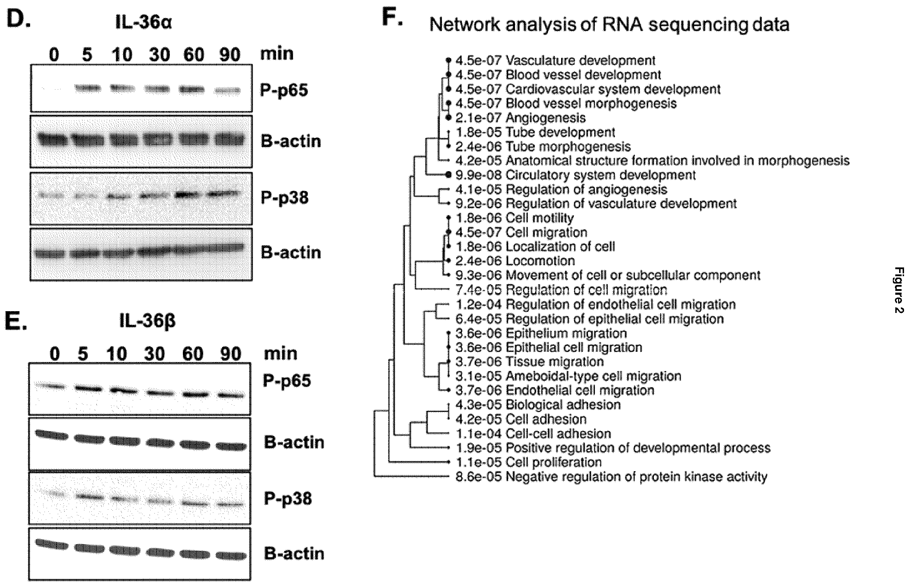

Figure 2 illustrates IL-36 Receptor expression and activation in microvascular endothelial cells, wherein (A) is brain endothelial cell heatmap depicting expression of actively translated IL-1 family receptors at baseline and in response to in vivo LPS challenge; (B) is relative expression of IL-36R and IL-1RAcP in HRMEC demonstrated via qPCR, with $\beta$-actin as a housekeeping gene and normalized to IL-18R expression, consolidated N=4, wherein statistical analysis was performed using t-tests to compare treatment group to untreated control, where * = p<0.05, **= p<0.01 *** = p<0.001; (C) is localization of IL-36R in HRMEC, representative images of N=3 experiments at 40 X magnification, (D&E) are Western blot demonstrating activation of MAPK and NF$\kappa$B in HRMEC in response to 60 ng/ml of (D) IL-36$\alpha$ (E) IL-36$\beta$ over a 90-minute time course, representative of N=3 experiments; (F) is network analysis of RNA sequencing data from HRMEC treated with 100 ng/ml IL-36$\beta$ for 24 hours, with pathway enrichment analysis for the 60 most differentially expressed genes showing 30 most significant terms using ShinyGO v0.61 comparing to GO biological processes, wherein P-value cut-off (FDR) is set to 0.05;

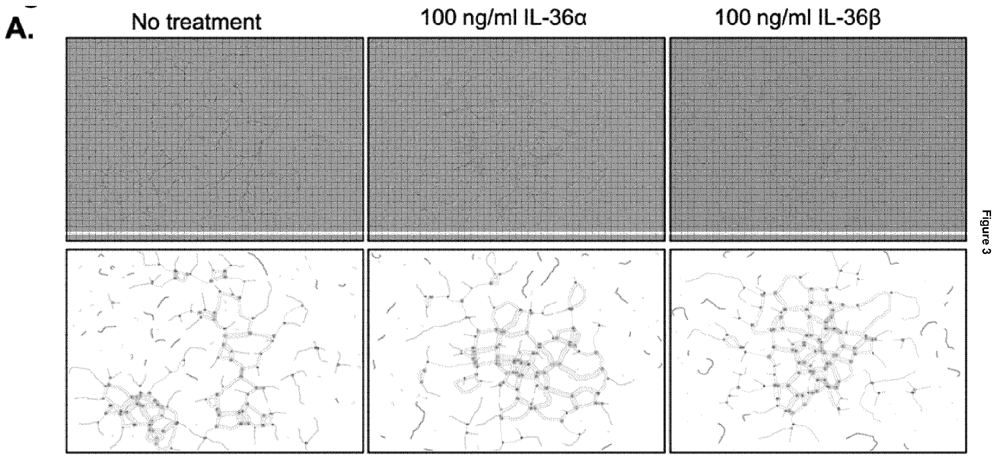

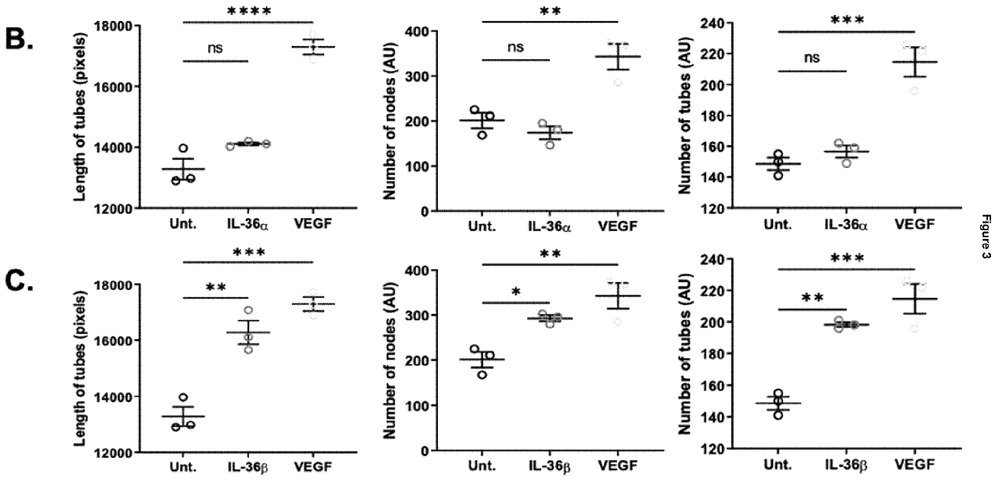

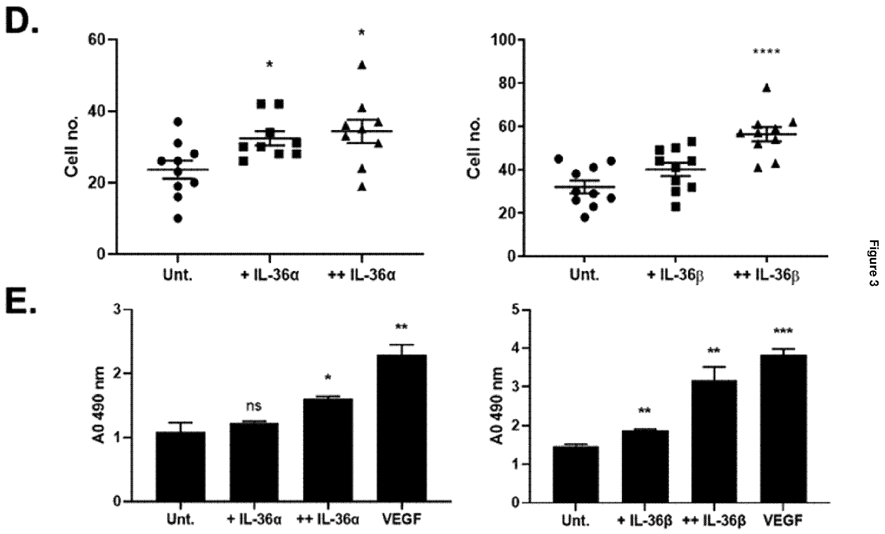

Figure 3 illustrates IL-36 cytokines influence tube formation and proliferation in endothelial cells, wherein (A) is representative phase contrast and analysed network images for no treatment, 100 ng/ml IL-36$\alpha$ and 100 ng/ml IL-36$\beta$

treatment at 4 hours after seeding HRMEC onto matrigel, wherein network analysis was performed using Angiogenesis Analyzer plug in for Image J, representative of N=5 experiments; (B, C) are analyses of number of tubes, number of nodes and tube length in HRMEC after 4 hours of growing in matrigel with 100 ng/ml (B) IL-36α (C) IL-36β treatment, with 50 ng/ml VEGF used as a positive control, representative of N=5 experiments; (D) is cell count of DAPI stained nuclei in HRMEC treated with 100 ng/ml IL-36α or IL-36βfor 24 hours, representative of N=3 experiments; (E) is MTS/PMS assay illustrating increase in proliferation with IL-36α or IL-36β (+ = 50 ng/ml, ++ = 100 ng/ml) treatments for 24 hours, with 24 hour treatment of VEGF at 50 ng/ml used as positive control, representative of N=3 experiments, wherein statistics were performed using one-way ANOVA with multiple comparisons; $p<0.05 = *$, $p<0.01 = **$, $p<0.005 = ***$, $p<0.001 = ****$;

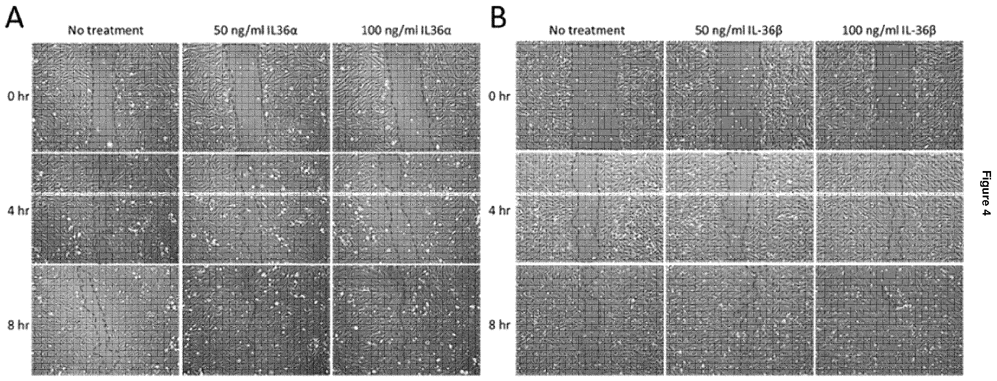

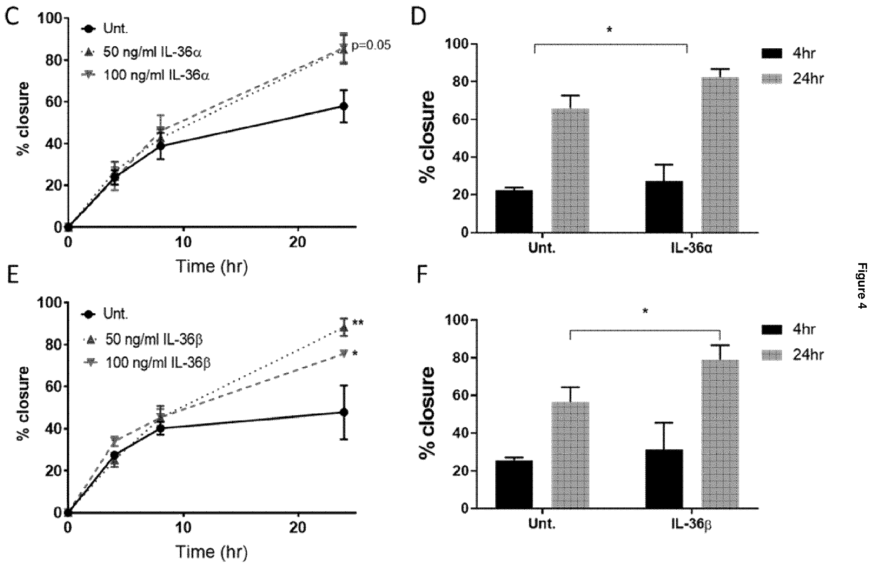

**Figure 4** illustrates IL-36 cytokines promote endothelial cell migration, wherein (A, B) are representational images of the HRMEC 'scratch' healing with (A) IL-36α (B) IL-36β treatment compared to control at 10X magnification, wherein purple dotted lines equate to the size of the wound as imaged using Image J; (C) is rate of wound healing in HRMEC treated with IL-36α expressed as % wound closure, representational of N=4 experiments, wherein two-way ANOVA was performed as statistical test; (D) is a comparison of the level of wound healing between untreated (unt.) and 100 ng/ml IL-36α at 4 and 24 hr post scratch, consolidated N=4 experiments, wherein two-way ANOVA with multiple comparison was performed as statistical test; (E) is rate of wound healing in HRMEC treated with IL-36β expressed as % wound closure, representational of N=4 experiments, wherein two-way ANOVA with multiple comparison performed as statistical test; (F) is a comparison of the level of wound healing between no treatment and 100 ng/ml IL-36β at 4 and 24 hr post scratch, consolidated N=4 experiments, wherein two-way ANOVA with multiple comparison was performed as statistical test;

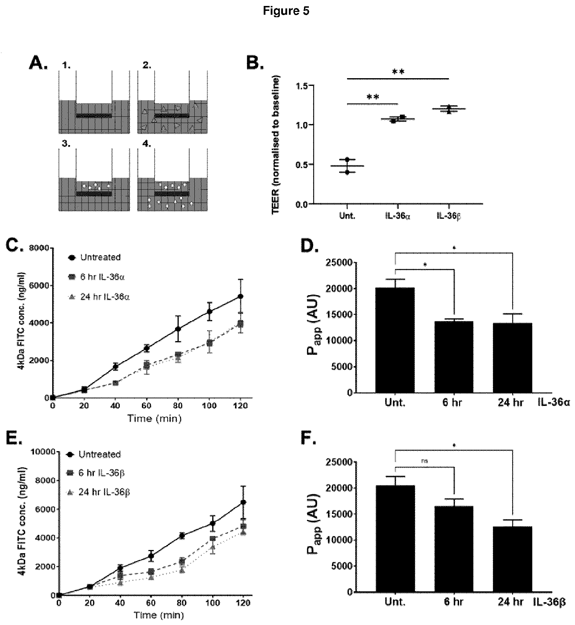

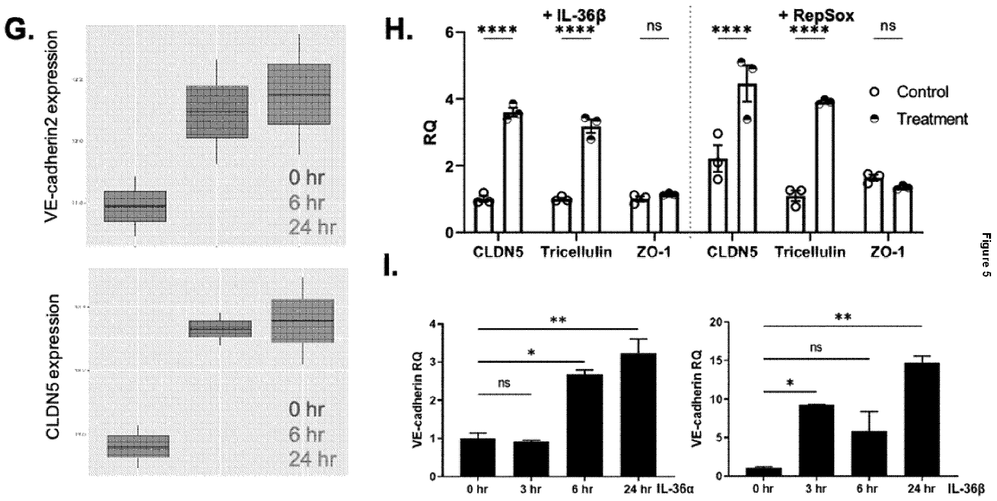

**Figure 5** illustrates IL-36 cytokines reduce the permeability of endothelial cell monolayer, wherein (A) is schematic illustration of FITC flux experiment, indicating (1) HRMEC seeded and allowed to form a monolayer in semi-permeable transwells prior to (2) treatment with cytokine in the apical and basal chambers followed by (3) FITC-dextran solution of known concentration replacing the media in the top chamber and (4) media periodically being sampled from the basal chamber to measure the rate of FITC flux; (B) is TEER reading of HRMEC transwell monolayer after 6 hr treatment of 100 ng/ml IL-36α or IL-36β, with blank well reading subtracted from TEER of experimental wells and blanked TEER readings post treatment normalised to blanked TEER readings taken of the same well before treatment, wherein one-way ANOVA with multiple comparisons was performed as statistical measure; (C) is rate of flux of 4 kDa FITC dextran through HRMEC transwell monolayer with 6 and 24 hour pre-treatment of IL-36α cytokine at 100 ng/ml, with treatment applied to apical and basal chamber; (D) is permeability apparent for HRMEC treated with 100 ng/ml IL-36α, calculated as Papp (cm/s) = (dQ/dT) / (A x C0), wherein T-tests were performed as statistical measure; (E) is rate of flux of 4 kDa FITC dextran through HRMEC transwell monolayer with 6 and 24 hour pre-treatment of IL-36β cytokine at 100 ng/ml, with treatment applied to apical and basal chamber; (F) is permeability apparent for HRMEC treated with 100 ng/ml IL-36β, calculated as stated previously with T-tests performed for statistical measure; (G) is box-plots of VE-cadherin 2 and cldn5 expression in HRMEC following treatment with 100 ng/ml IL-36β, highlighted as differentially expressed genes using iDEP to analyse gene counts and identify differential expression; (H) is qPCR for cldn5, tricellulin and ZO-1 expression performed with RNA extracted from HRMEC treated with 100 ng/ml IL-36β or 10 μM RepSox for 24 hours, normalised to untreated control and β-actin used as housekeeping gene, wherein two-way ANOVA was performed as statistical test; (I) is qPCR for expression of VE-cadherin mRNA in HRMEC in response to 100 ng/ml IL-36α or IL-36β treatment over a 24-hour timecourse, normalised to 0 hr with β-actin used as housekeeping gene, wherein one-way ANOVA was used as statistical measure, wherein all data are representative of N=3 experiments, excluding G which is representative of N=2 samples;

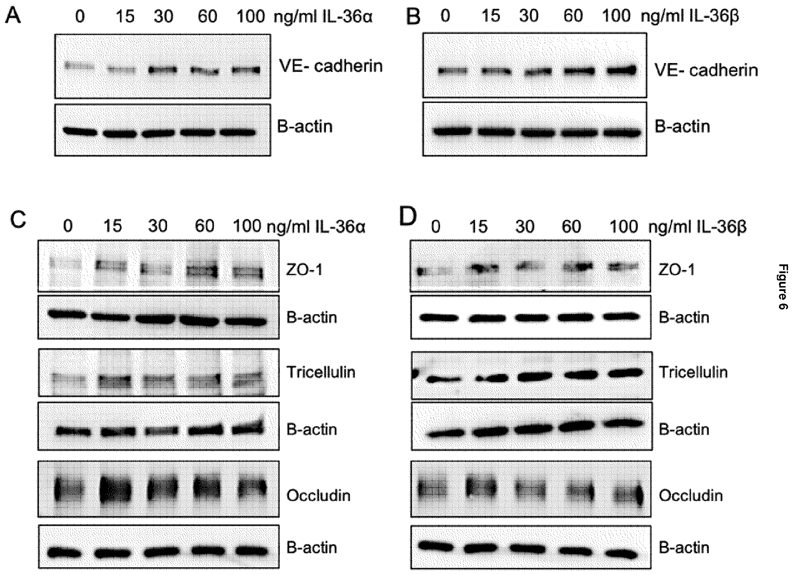

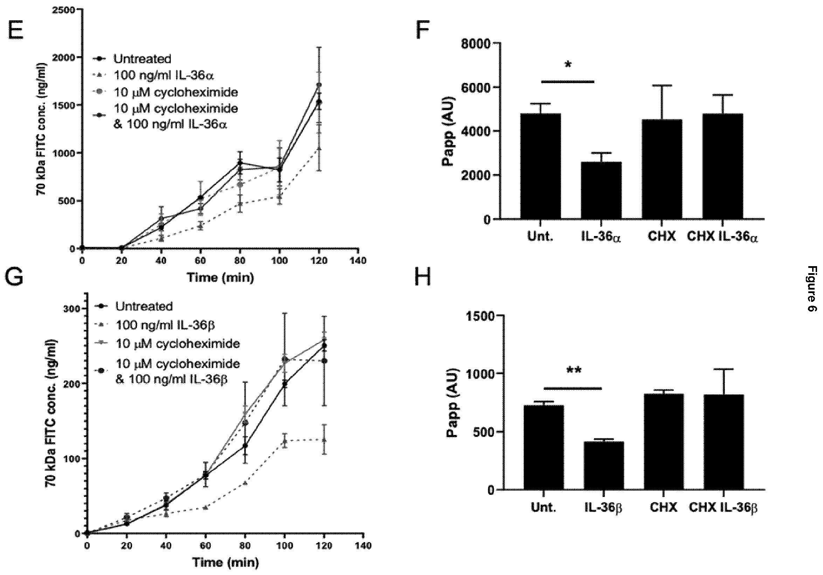

**Figure 6** illustrates IL-36 cytokines alter the protein levels of adherens and tight junction components in endothelial cells, wherein (A, B) are Western blot for VE-cadherin protein expression in response to (A) IL-36α, (B) IL-36β treatment for 24 hours at indicated doses in HRMEC, representative N=4 experiments; (C, D) are Western blot for the expression of tight junction proteins ZO-1, tricellulin and occludin in response to (C) IL-36α and (D) IL-36β treatment at indicated concentrations for 24 hours in HRMEC, representative of N=3 experiments; (E) is rate of flux of 70 kDa FITC dextran through HRMEC transwell monolayer with 6 hr pretreatment of 100 ng/ml IL-36α and/or 10 μM cycloheximide (CHX), with treatment applied to apical and basal chambers, representative of N=3 experiments; (F) is permeability apparent for HRMEC treated with IL-36α and/or 10 μM CHX, calculated as stated previously; (G) is rate of flux of 70 kDa FITC dextran through HRMEC transwell monolayer with 6hr pretreatment of 100 ng/ml IL-36β and/or 10 μM CHX, with treatment applied to apical and basal chambers, representative of N=3 experiments; (H) is permeability apparent for HRMEC treated with IL-36β and/or CHX, calculated as stated previously, wherein T-tests were performed as statistical analysis;

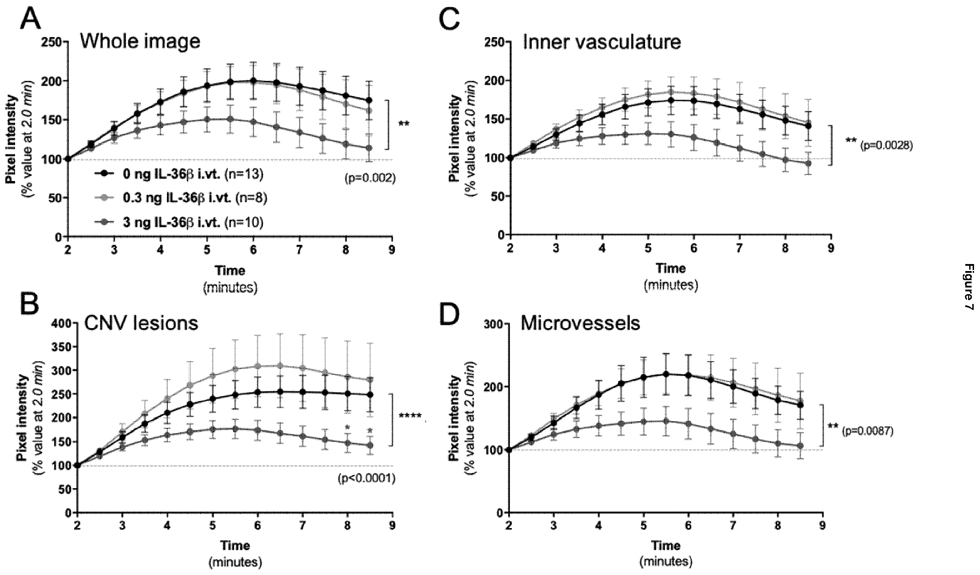

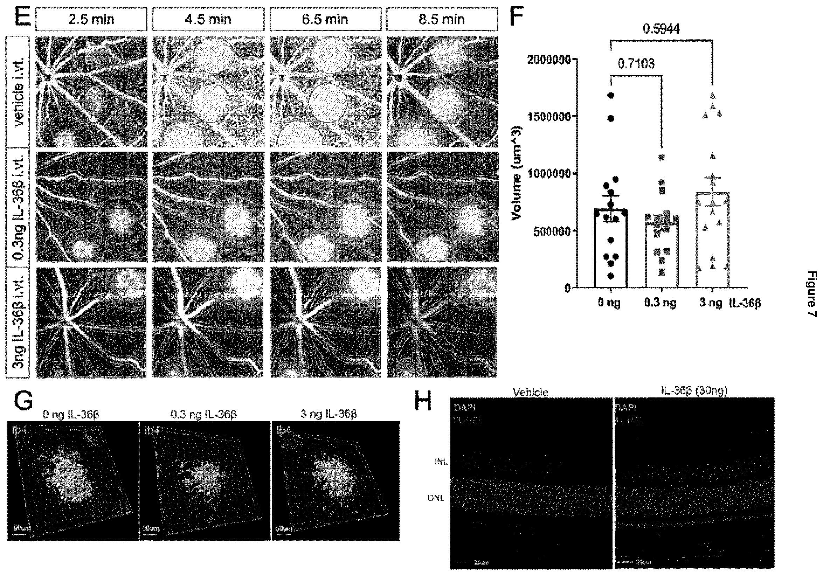

**Figure 7** illustrates IL-36β decreases BRB permeability without affecting neovascular lesion size in vivo; wherein (A-D) are dynamic FA profiles acquired in C57BL/6J mice 5 days after the administration of laser-induced CNVs and intravitreal IL-36β (0.3 and 3 ng) or vehicle control (0 ng), normalised signal intensity quantified in the overall image (A), at the site(s) of CNV (B), inner vasculature outside the CNV-affected area (C) or microvessels (D); (E) is representative FA angiograms at 2.5, 4.5, 6.5 and 8.5 minutes post fluorescein injection for 0, 0.3 and 3 ng IL-36β with different regions of interest highlighted; (F) is volumetric analysis of CNV lesions based on GS-IB4 isolectin 6 days after laser-induced CNV administration and IL-36β treatment (n=15 for vehicle and 0.3 ng IL-36β groups, n=17 for 3 ng IL-36β group), wherein volumetric analysis was performed blinded, wherein each CNV lesion was assessed prior to inclusion in volumetric analysis, with all haemorrhages excluded, wherein statistics were determined using ANOVA with multiple comparisons; (G) is representative 3D renders of GS-IB4 isolectin-based CNV volumes for 0, 0.3 and 3 ng IL-36β; (H) is TUNEL staining of paraffin sections taken from C57BL/6J mice 3 days post intravitreal injection with PBS or 30 ng IL-36β;

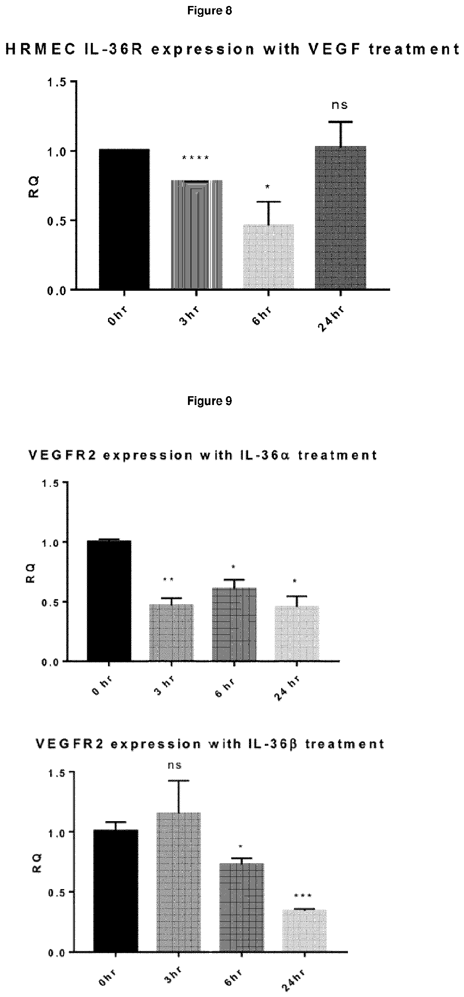

**Figure 8** illustrates VEGF regulates IL-36R expression signalling, wherein HRMECs were treated with VEGF over timepoints indicated and cells were prepared for qPCR, wherein expression levels of IL-36R were measured by ddCT method, wherein data means ± SD are representative of n=3 independent experiments;

**Figure 9** illustrates IL-36 cytokines regulate VEGFR2 expression, wherein HRMECs were treated with either IL-36alpha or IL-36beta over timepoints indicated and cells were prepared for qPCR, wherein expression levels of VEGFR2 were measured by ddCT method, wherein data means ± SD are representative of n=3 independent experiments;

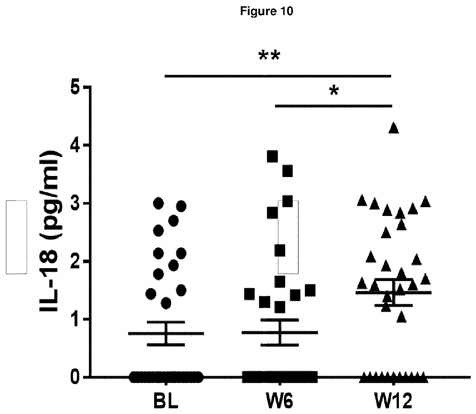

**Figure 10** illustrates IL-18 levels increased significantly over 12 weeks in patients with wet AMD given anti-VEGF treatment; wherein IL-18 levels were measured in aqueous humor of 33 AMD cases at 6-week intervals (BL, W6, W12) and 13 cataract controls, wherein baseline and week 6 levels of IL-18 were significantly lower than cataract controls (A), with a significant change in aqueous IL-18 from baseline to week 12 and week 6 to week 12 (B), wherein data were analysed using Kruskal-wallis test with Dunn's multiple comparisons test, wherein all data are presented using mean ±SEM (*p = <0.05);

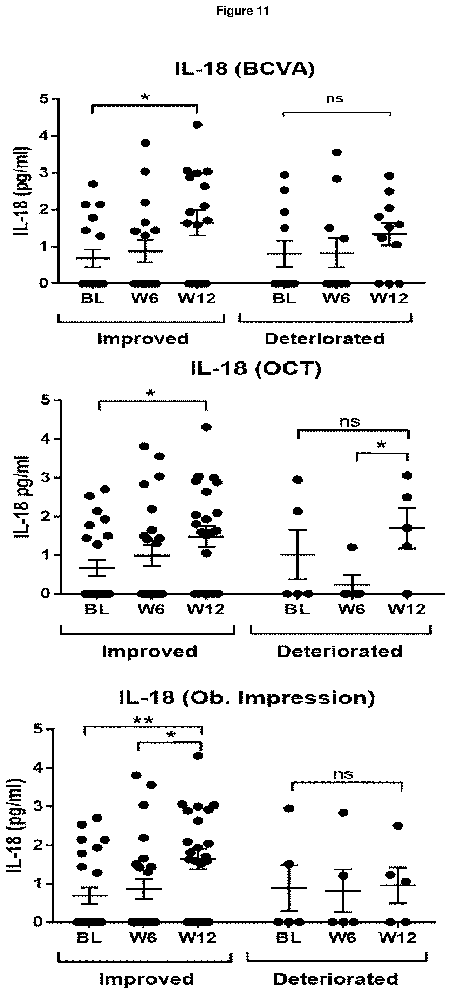

**Figure 11** illustrates AMD patients who respond to anti-VEGF therapy have a significant increase in IL-18 in their aqueous humor over 12 weeks, wherein IL-18 was measured in samples taken during the course of anti-VEGF treatment and participants were grouped based on response. IL-18 was found to increase significantly between baseline and week 12 in participants who had improved BCVA (A), OCT (B) and objective impression which also had a significant rise between week 6 and week 12 (C), wherein only those who did not respond as based on OCT measurement had a significant change in IL-18 levels during treatment, wherein data are analysed by two-way ANOVA with Tukey's multiple comparison test, wherein all data are presented using mean±SEM (*p = <0.05, **p = <0.01, ns = not significant);

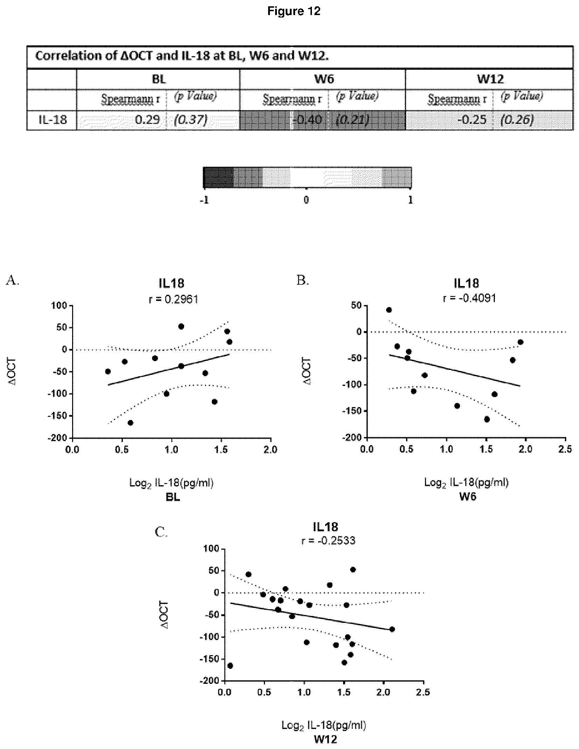

**Figure 12** illustrates correlation between overall change in OCT from pre- to post-treatment, wherein correlation analysis of change in CMT (ΔOCT) and IL-18 levels at baseline (n=11) **(A)**, week 6 (n=11) **(B)** and week 12 (n=21) **(C)** in a clinical neovascular cohort, wherein data were analysed using Spearman's correlation with graphs showing 'r' coefficient;

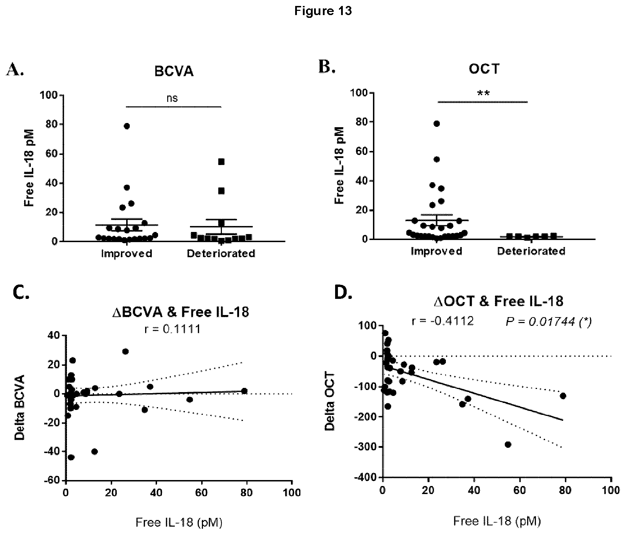

**Figure 13** illustrates plasma IL-18 correlates with reduced retinal oedema, wherein participants were grouped based on functional outcome measurements post treatment and found that there was no difference in baseline IL-18 levels between those who improved or deteriorated based on BCVA (A), however plasma IL-18 at baseline was significantly higher in those who had improved OCT measurement post treatment (B), wherein correlation analysis found no significant association with baseline IL-18 levels and overall change in BCVA (C), but higher levels of IL-18 at baseline correlated with a greater decrease in retinal thickness during treatment (P=0.01) (D), wherein data are analysed using Mann-Whitney t-test between groups and Spearman's rank correlation, wherein data are presented using mean ±SEM (* p <0.005, ** p =<0.001, ns = not significant);

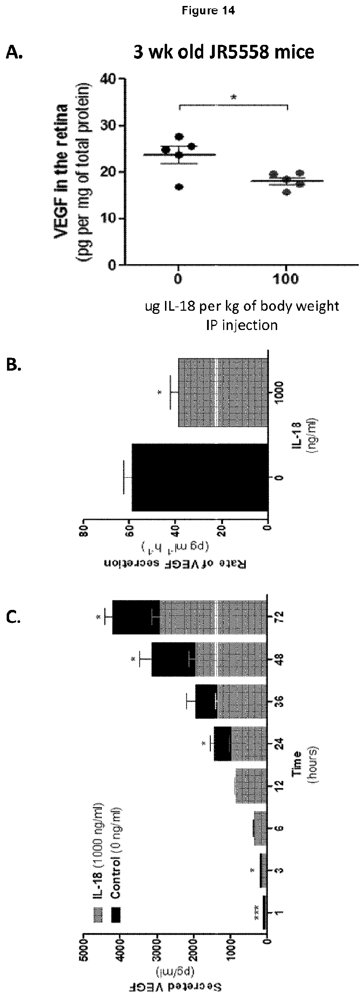

**Figure 14** illustrates IL-18 can inhibit VEGF secretion in vivo and in vitro, wherein JR5558 mice at 3 weeks (A) were injected (i.p.) with saline (0 μg/kg) or IL-18 (100 μg/kg) on 4 consecutive days (day 1-4), wherein retinas were isolated on day 8 and VEGF concentration in protein lysates was determined using an appropriate ELISA, wherein data are presented as mean ± SEM (n=4-5 animals per each treatment group) and expressed as pg of VEGF per mg of total retinal protein as measured by the BCA assay, *p<0.05 by one-way ANOVA with Tukey post test compared to saline controls; (B) is primary human foetal RPE (hfRPE) cells were treated with control medium (0 ng/ml) or a single dose of IL-18 (1000 ng/ml) for 1 to 72 hours, wherein VEGF concentration in the cell-free supernatants was measured at each time point, wherein rate of VEGF secretion was calculated from the slope of linear regression of VEGF concentration (pg/ml) against time (hours) (C), wherein data are presented as mean $\pm$ SEM and represent a single experiment carried out in triplicate *p<0.05, ***p<0.001 by Student's t test compared to control at each time point;

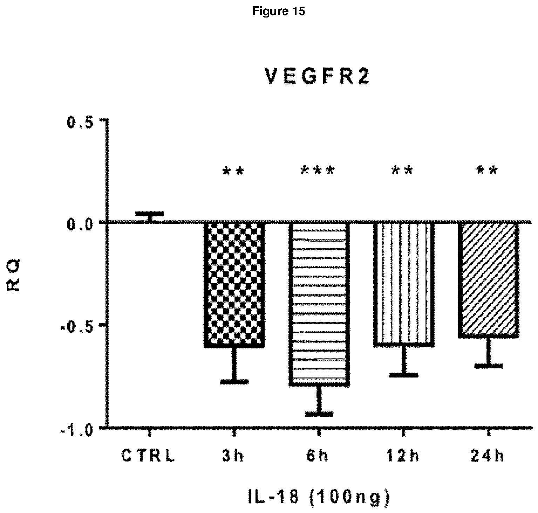

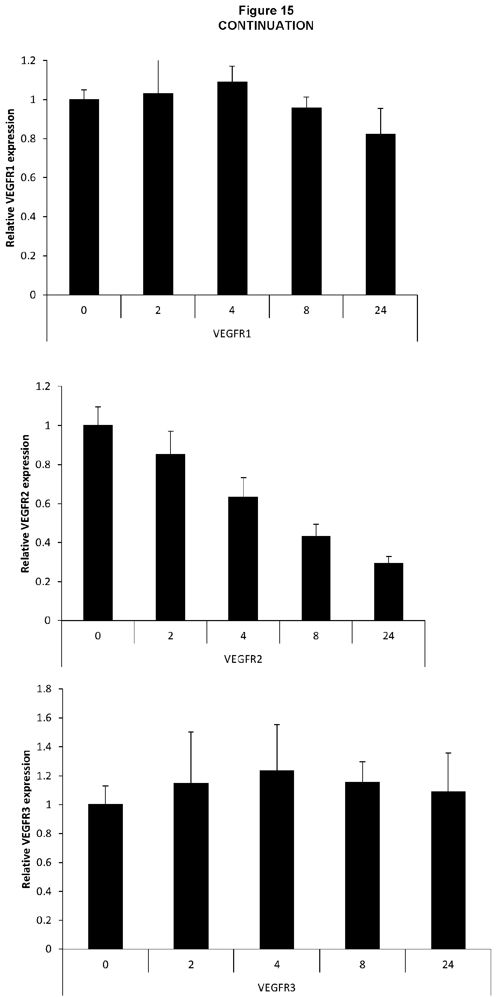

**Figure 15** illustrates IL-18 reduces expression of VEGFR2 specifically in Human retinal endothelial cells (HRMECs), wherein HRMECs were treated with IL-18 (100ng/ml) over timepoints indicated, wherein cells lysates were prepared for RNA isolation and underwent qPCR and ddCt method to assess relative expression of VEGFR levels, using B-actin as reference control, wherein RT-PCR data are means $\pm$SEM of n=3 independent experiments;

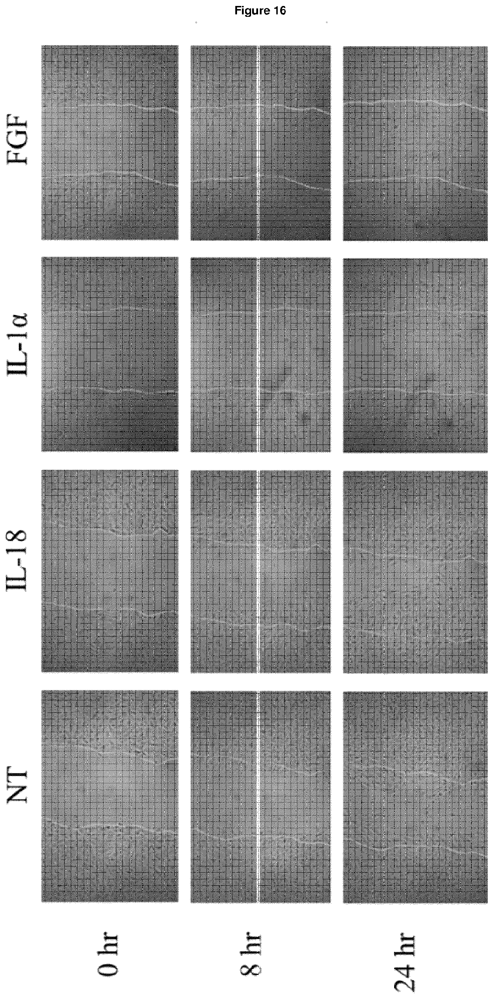

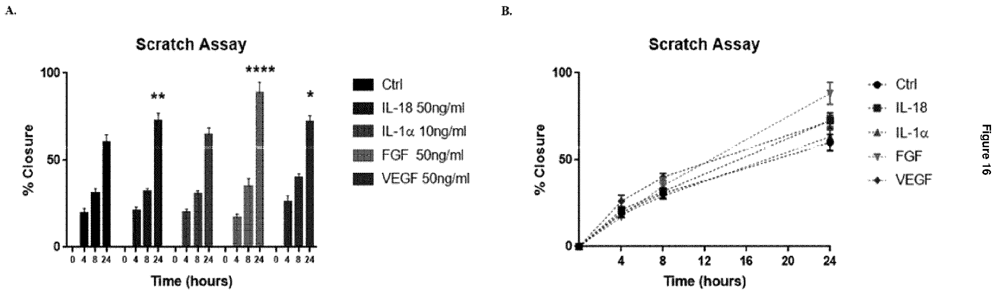

**Figure 16** illustrates HRMEC migration in response to IL-18 and IL-1$\alpha$ stimulation, wherein confluent HRMEC monolayers were scratched with a pipette tip and treated with IL-18 (50ng/ml) or IL-1$\alpha$ (10ng/ml) along with bFGF (50ng/ml) or VEGF (50ng/ml) as positive controls, wherein images were taken immediately following scratch (0 hrs) and at 4, 8 and 24 hours following scratch, wherein images were analysed using ImageJ imaging software, wherein data are presented as mean $\pm$SEM of 6 separate experiments in triplicate, wherein data were analysed using 2way ANOVA with Dunnett's multiple comparison test. (* p <0.05, * p <0.01, **** p <0.0001);

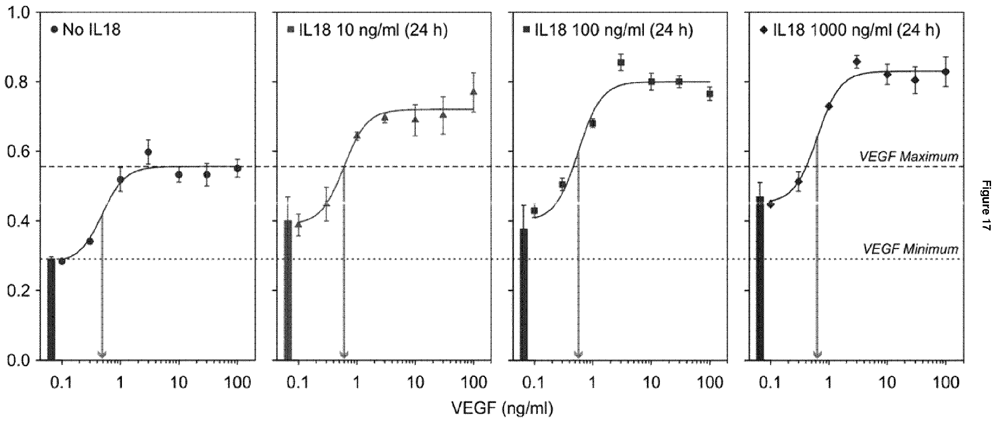

**Figure 17** illustrates HRMEC proliferation in response to VEGF is enhanced with IL-18 pre-treatment, wherein HRMECs were treated with increasing concentrations of VEGF alone or with a 24 hour pre-treatment of IL-18 at 10, 100 or 1000 ng/ml, wherein increasing VEGF induced an increase in cell proliferation which was enhanced following IL-18 treatment;

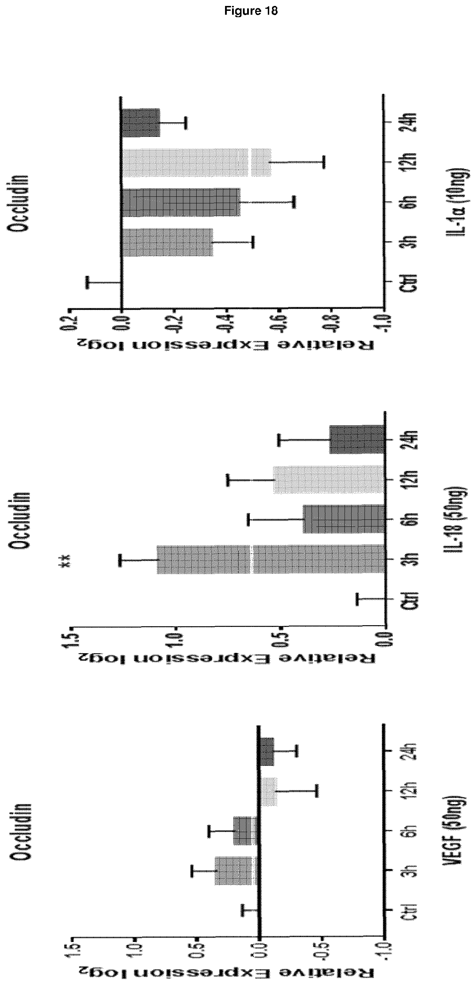

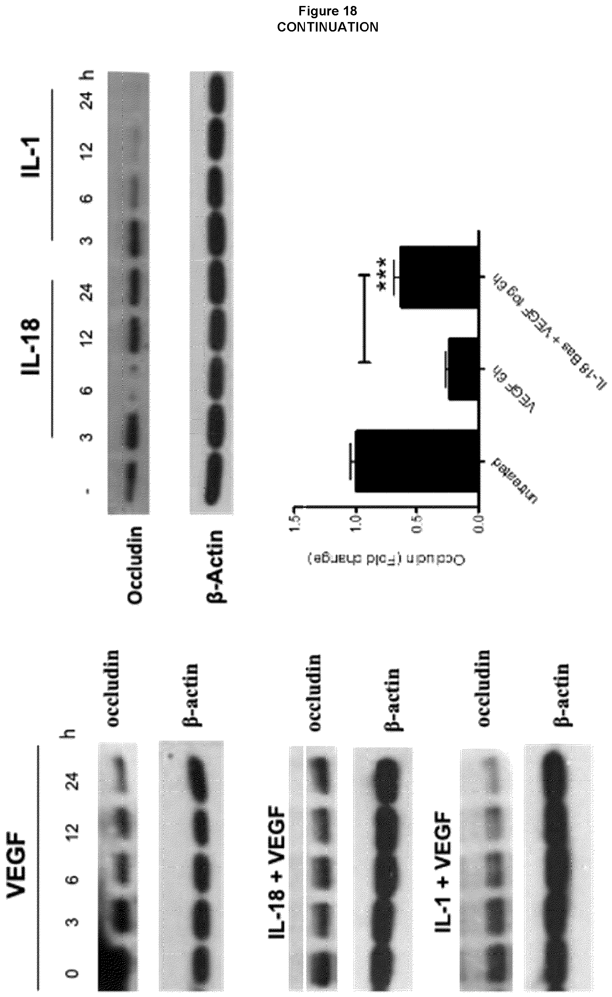

**Figure 18** illustrates IL-18 drives expression of tight junction protein occludin and inhibits VEGF depletion of occluding, wherein HRMECS were treated with either VEGF, IL-18 or IL-1a for the timepoints indicated (top panel) and cells were prepared for RNA isolation and qPCR with expression of occluding measured, wherein HRMECs were treated with VEGF, IL-18 or IL-1 or combinations of these cytokines and cells were prepared for immunoblotting for expression of occludin and b-actin (lower panels);

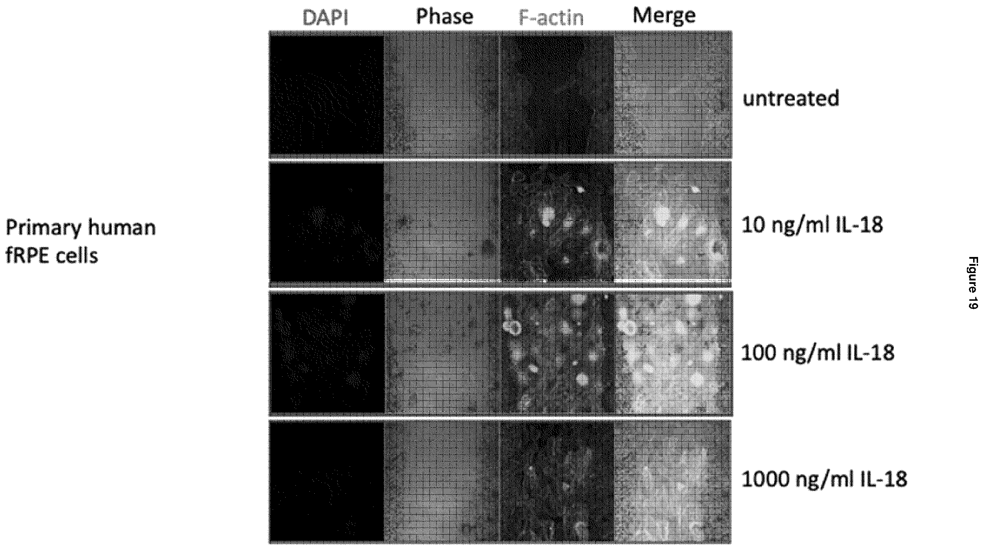

**Figure 19** illustrates IL-18 induces fibroblast-like cells to migrate to close a wound created in human Retinal Pigment Epithelium monolayer in vitro, wherein primary human foetal RPE (hfRPE) cells were treated with control medium (0 ng/ml) or increasing doses of IL-18 (10-1000 ng/ml) immediately after making a scratch through the centre of the well, wherein microscopy images were acquired at the same location at baseline (0 hours) and then every 2 hours until 18 hours, representative images for each treatment group are shown, wherein cells were stained with DAPI to show the nuclei, and phalloidin to show F-actin, wherein Brightfield images (phase) are also shown;

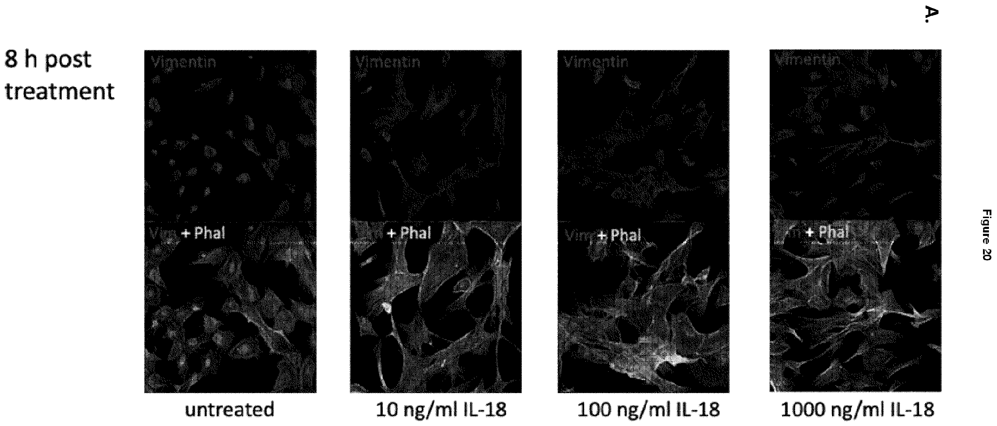

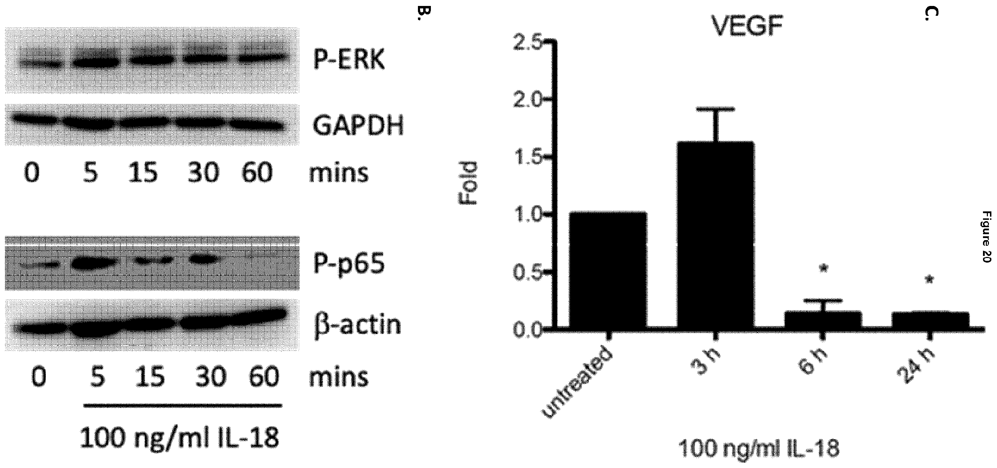

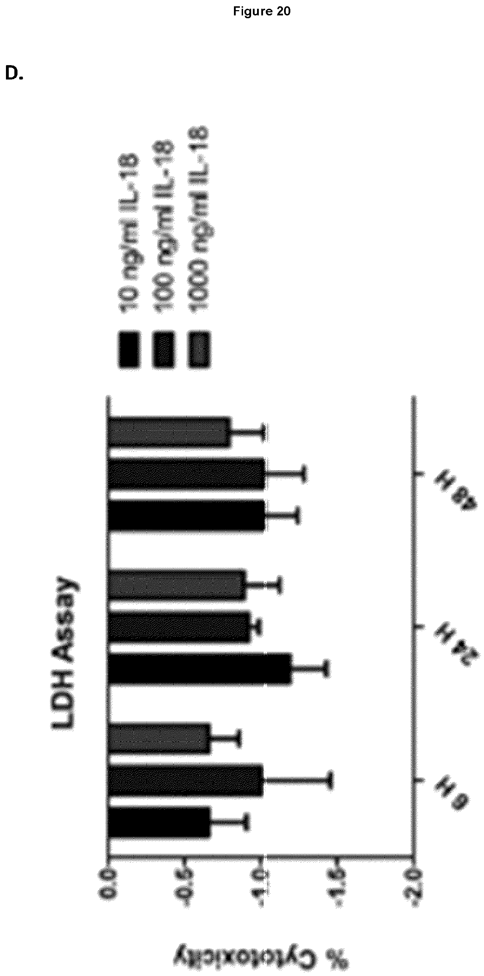

**Figure 20** illustrates IL-18 activates human ocular choroidal fibroblasts and inhibits VEGF secretion, wherein (A) is human ocular choroidal fibroblast (HOCFs) treated with IL-18 at increasing doses for 8h, wherein cells were fixed and stained with antibody targeting intermediate filament vimentin (red), Hoechst (blue) and phalloidin (green); (B) is HOCFs treated with IL-18 (100 ng/ml) over 60 mins and immunoblotted for phospho-ERK and phospo-p65 with B-actin and GAPDH as controls; (C) is HOCFs treated with IL-18 (100 ng/ml) over 24 h as indicated, wherein cells were analysed for VEGF expression by qPCR (D) is HOCFs treated with 10, 100 or 1000 ng/ml of IL-18 as indicated over 48 h and cell death was analysed by LDH assay;

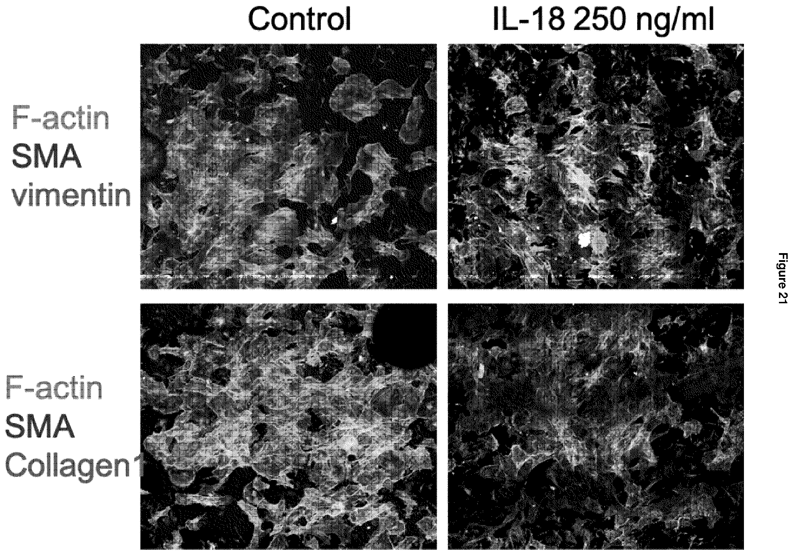

**Figure 21** illustrates IL-18 activates bone marrow derived mesenchymal stem cells, wherein bone marrow derived MSC's were treated with IL-18 at 250ng/ml for 8 h and images using confocal microscopy, wherein cells were stained with phalloidin for F-actin, vimentin, smooth muscle actin (SMA) and collagen 1;

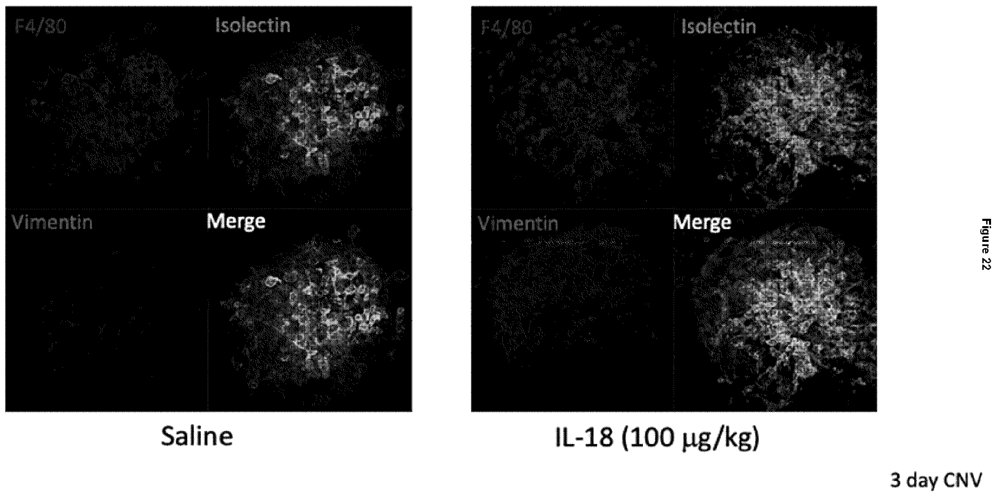

**Figure 22** illustrates increased mesenchymal cell infiltration/activation in animals injected with IL-18 intrperitoneally, wherein WT mice underwent laser induced CNV and were injected IP with IL-18 (100ug/kg), wherein 3 days later eyes were enucleated, flatmounts were stained with F4/80, vimentin and isolectin and imaged by confocal microscopy;

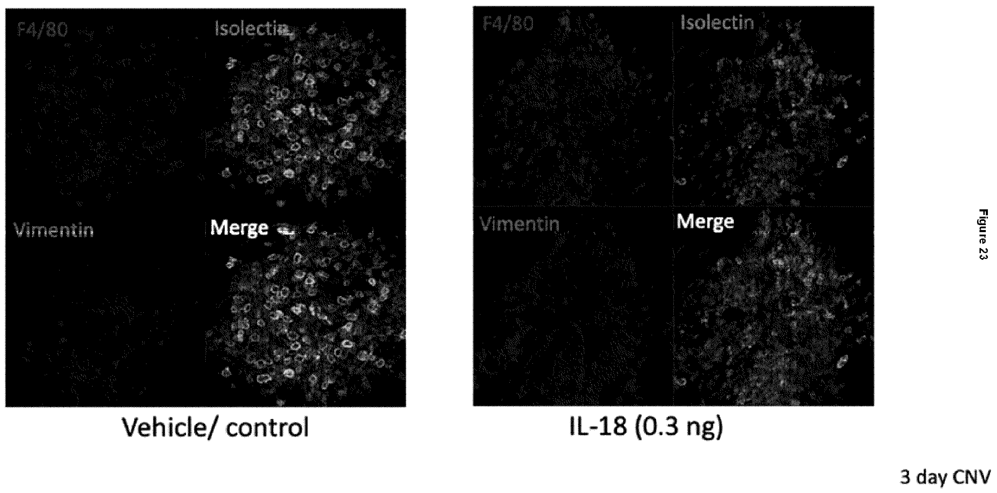

**Figure 23** illustrates increased mesenchymal cell infiltration/activation in animals injected with IL-18 intra-vitreally, wherein WT mice underwent laser induced CNV and were injected intravitreally with IL-18 (0.3ng/eye), wherein 3 days later eyes were enucleated, flatmounts were stained with F4/80, vimentin and isolectin and imaged by confocal microscopy;

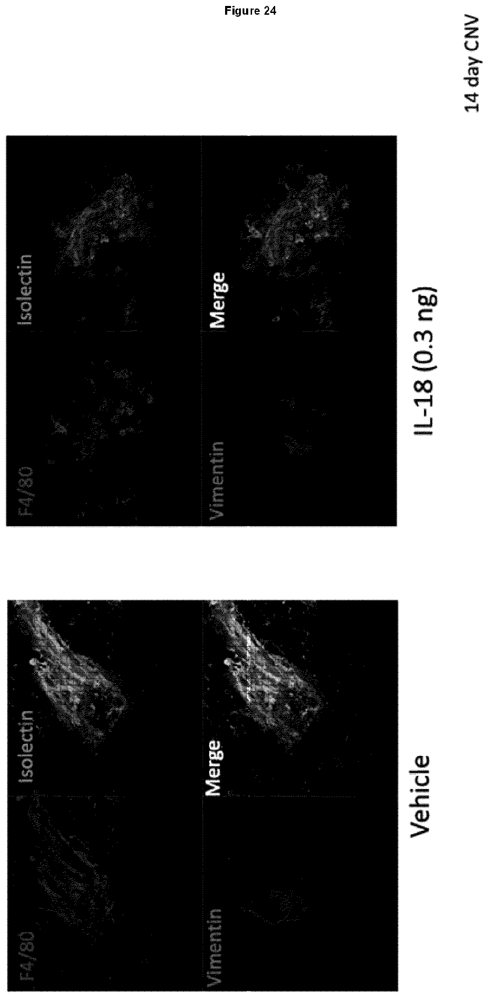

**Figure 24** illustrates IL-18 reduces the area of fibrovascular scarring, wherein WT mice underwent laser induced CNV and were injected intravitreally with IL-18 (0.3ng/eye), wherein 14 days later eyes were enucleated, flatmounts were stained with F4/80, vimentin and isolectin and imaged by confocal microscopy; and

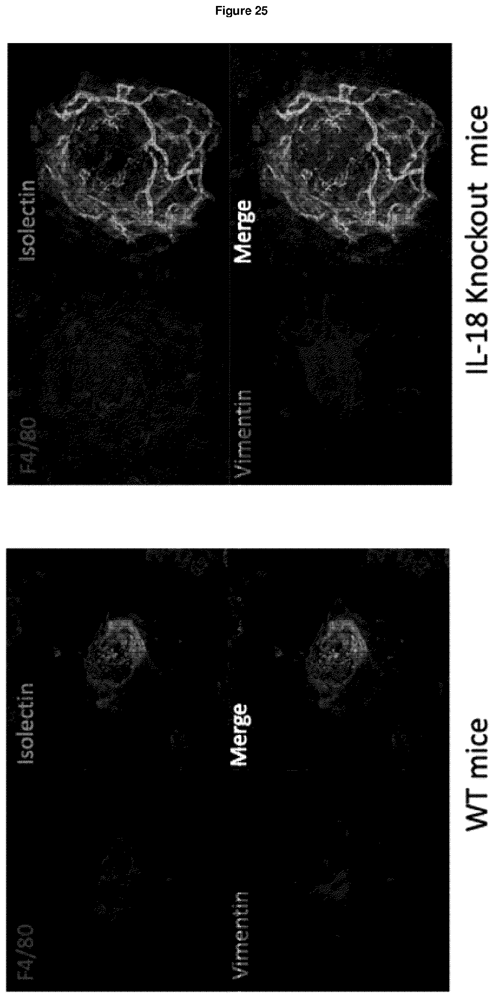

**Figure 25** illustrates IL-18 deficiency increases the area of fibrovascular scarring, wherein WT and IL-18 KO mice underwent laser induced CNV, wherein 14 days later eyes were enucleated, flatmounts were stained with F4/80, vimentin and isolectin and imaged by confocal microscopy.

## Examples

## Materials and Methods

### Study Design

The goals of this study were to determine the roles of IL-36 in angiogenic processes and vascular permeability in vivo and in vitro. FFA was used to determine permeability in vivo after laser injury, with treatment conditions blinded during assessment of outcomes. Human retinal microvascular endothelial cells were used to model the effects of IL-36 on angiogenic processes and barrier integrity in vitro, as a primary interest was how IL-36 acts in the CNS, in particular the iBRB, which these cells are a major constituent of in vivo. Experimental numbers, biological replicates and sample sizes for each experiment are outlined in the figure legends.

### Retinal flatmount analysis

Eyes were enucleated from 12-month old wild-type C57BL/6J and IL-36rn-/- mice (received from M.Kopf -Zurich (Tortola L, Rosenwald E, Abel B, Blumberg H, Schäfer M, Coyle AJ, Renauld JC, Werner S, Kisielow J & Kopf M (2012) Psoriasiform dermatitis is driven by IL-36-mediated DC-keratinocyte crosstalk. J Clin Invest 122: 3965-3976) and fixed in 4% PFA for 15 minutes before the sclera and lens were removed and eye cups were given 4 radial cuts. Eye cups were incubated with a Griffonia-simplicifolia-isolectin-Alexa 488 (Thermo Fisher) (1:500) overnight at 4oC and then mounted. Retinal flatmounts were imaged using a Leica SP8 scanning confocal to capture Z-stack images. These images were flattened and analysed using the REAVER program. Additionally image J was used to quantify MFI of the flattened Z-stack images.

### Cell culture

Primary human retinal microvascular endothelial cells (HRMEC) (Innoprot) were seeded into tissue flasks coated in fibronectin (Sigma). HRMEC were cultured in endothelial cell basal medium (PromoCell) containing 5% FCS, growth factor supplement and 1% Pen-strep (Sigma), henceforth known as growth medium. When experimental procedure required that the cells be serum deprived, media with no growth factor and 0.5% FCS was used. Cultures were incubated at 37°C, 5% $CO_2$ and 95% relative humidity. All cells used for experimentation were between passages 4 and 8. Cells were treated with various concentrations of recombinant human (rh) IL-36$\alpha$, IL-36$\beta$ and VEGF (all Peprotech) for experiments.

### Real-time qPCR

Total RNA was extracted from HRMEC using EZNA kit (Omega) and was reversed transcribed using M-MLV transcriptase (Promega) as per manufacturer's protocol. The cDNA was used as a template for target gene amplification, along with the housekeeping gene $\beta$-actin, by Real Time PCR using SensiFAST SYBR Green (Bioline) to determine the relative amounts of the genes of interest. The 2-$\Delta\Delta$CT method was used to calculate relative gene expression. Primers used were sourced from Sigma.

### RNA sequencing

HRMEC were treated with IL-36$\beta$ for 24 hours and RNA was extracted post-treatment as described above. mRNA enrichment was performed on 100 ng of total RNA using the NEBNext Poly(A) mRNA magnetic isolation module. Libraries were prepared using NEBNext Ultra II RNA Library Prep Kit for Illumina and sequenced using a NextSeq 500 output kit v2.5 (Illumina) with the Illumina NextSeq 500 platform. Sequence analysis was performed on MacOS using Python 3.8.5 and all steps were performed in paired-end mode where applicable. Adapter trimming and quality control was performed using cutadapt 3.1 with a minimum posttrimming length of 21 nucleotides and using TruSeq adapter sequences. Alignment was performed with HiSAT2 version 2.2.1, using an index built from Gencode version 37 (GRCh38.p13) of the human genome. Samtools version 1.10 was used to sort and index the aligned reads. For differential gene expression analyses, Wald testing was performed in featureCounts version 2.0.1. For further downstream analyses, the iDEP 9.2 online tool was used to generate PCA plots, perform hierarchical clustering and gene expression pathway analyses. Alignments were visualised using the integrated genomics viewer (IGV).

### Immunocytochemistry

HRMEC were grown in chamber slides until confluent. They were washed in PBS, fixed in ice cold methanol and blocked/permeabilised in 5% NGS/0.05% Triton X-100 made up in PBS for 30 minutes. Primary antibody (IL-36R Novus Biologicals, VE-cadherin Santa Cruz, IL-1RAcP Invitrogen) was applied overnight and incubated at 40°C. Cells were then washed in PBS before being incubated in secondary antibody for 2 hours at room temperature. Following this incubation, the slides were washed with PBS, stained with Hoechst for 2 minutes before being washed again, chambers removed and mounted with coverslips. The slides were then imaged at 40X using a Zeiss confocal microscope.

### Western Blotting

Protein was extracted from cells using RIPA buffer containing protease inhibitors (Sigma). Sample buffer was added to protein samples and denatured at 100°C for 10 minutes. Samples were then resolved by SDS-PAGE and transferred to PVDF membrane (Sigma). Membranes were blocked with 5% skimmed milk (Marvel) in TBST. Membranes were incubated overnight at 4°C with primary antibody diluted in 5% skimmed milk in TBST. Anti-Rabbit or anti-mouse secondary antibody (Sigma) was applied to the membrane as was appropriate and incubated at room temperature for an hour. Blots were then developed using Western Bright ECL (Advansta). Densitometry on blots was performed using the Image J software.

### Proliferation assays

HRMEC were seeded on coverslips in 6 well plates at a density of 5 x 104 cells/ml and allowed to grow overnight in growth media. They were then washed in PBS and placed in reduced serum media and incubated with treatment for 24 hour. The coverslip was then washed in PBS, fixed in ice cold methanol and stained with Hoechst (1:10000). The coverslips were then affixed to slides and imaged via fluorescence. N=10 images per slip were taken and averaged to give a cell count per coverslip. For the MTS/PMS assay (Promega) cells were seeded at a density of $5 \times 10^4$ cells/ml in a 96 well plate. They were allowed to grow overnight in complete growth media before being washed and placed in low-serum media with the treatment indicated for 24 hours before the MTS/PMS assay was carried out as per manufacturer's protocol.

### Tube formation assay

Cells were seeded 2 days prior to the experiment in a T75 in complete growth media and allowed to become ~80% confluent. They were placed in low serum media overnight prior to use in the experiment. 96 well plates were coated with 60 $\mu$l of Matrigel Basement Membrane Matrix (Corning) and placed in a 370C incubator for 30 minutes to set as per manufacturer's instructions. HRMEC were then seeded onto the plates at a density of $8 \times 10^4$ cells/ml and treated as indicated with cytokine in triplicate. The plates were placed in an Incucyte and phase contrast images of the wells were taken at 10X for 30 minute intervals. The angiogenesis analyser plug-in for ImageJ was used to analyse the images.

### Permeability assay

HRMEC were grown on transwells (Corning) for 5-7 days to polarize. The cells were placed in low serum media and treated with IL-36 cytokines for 6 or 24 hours, before 4kDa FITC dextran (Sigma) diluted to 1 mg/ml in low serum media was placed in the apical chamber. Sampling aliquots were periodically removed from the basal chamber and placed in a 96 well plate. The basolateral chamber was then replenished with fresh media. This was repeated at 20-minute intervals for 2 hours. The fluorescence of FITC dextran was measured using a fluorescent plate-reader (BioTek) at an excitation wavelength of 485 nm and an emission wavelength of 520 nm. Relative fluorescence units (RFU) were converted to ng/ml using a FITC dextran standard curve and corrected for background fluorescence and serial dilutions over the course of the experiment. The apparent permeability coefficient (Papp) was calculated as follows:

$$P_{app} \text{ (cm/s)} = (dQ/dT) / (A \times C_0)$$

where dQ/dT is the apparent rate of FITC dextran appearance in the basolateral chamber, A ($cm^2$) is the surface area of the insert, $C_0$ (mg/ml) is the initial FITC-dextran concentration in the apical chamber. dQ/dT is the slope (y = mx + c) calculated by plotting the cumulative amount (Q) against time(s).

## Trans-endothelial electrical resistance (TEER) measurement

HRMEC were grown in transwells as described previously. They were treated as indicated with IL-36 cytokine in both apical and basal chambers. An EVOM2 epithelial voltmeter with STX2 chopstick electrodes (World Precision Instruments) was used to measure the TEER of each transwell, with measurements taken in ohms and accounting for the nascent resistance of the transwell membrane by taking a measure of a well in culture media that had no cells.

## Inhibition of translation

HRMEC were grown in transwells as above. Once polarised, HRMEC were treated with 10 $\mu$M of the translation inhibitor cycloheximide (Sigma) and/or IL-36 cytokine. 70kDa FITC dextran (Sigma) diluted to 1 mg/ml in low serum media was placed in apical chamber and performing a FITC flux assay as described above.

## Laser-induced CNVs and intravitreal injections

All relevant national and institutional approvals were obtained prior to commencing experiments. 12 week old wild-type C57BL/6J mice had CNV induced using a green 532-nm Iridex Iris laser (532 nm, 140 mW, 100 ms, 50-mm spot size, three spots per eye) as described. After laser burn, 3 $\mu$l of vehicle or mIL-36$\beta$ was injected intravitreally at concentrations of 100 ng/ml or 1000 ng/ml. Mice were sacrificed 6 days post laser burn.

## RPE flatmount staining and CNV volume analysis

Eyes were enucleated and neural retina removed, the eye cups were given 4 radial cuts, avoiding the laser burns. The eye cups were incubated with anti-CD-31 antibody (Abcam) at 4°C overnight, then subsequently incubated with goat anti-rat IgG Alexa 594 (Abcam) and a Griffonia-simplicifolia-isolectin-Alexa 488 (Thermo Fisher) (1:400 in phosphate buffered saline) for 2 hours at room temperature temperature before mounting. CNV was assessed by confocal microscopy, and IMARIS software (Oxford instruments) was used to create renders from Z-stack images and calculate CNV volume.

## Acquisition of dynamic Fundus Fluorescein Angiography Images

Fundus fluorescein angiography was carried out using the Heidelberg Spectralis® HRA+OCT imaging platform (Heidelberg Engineering). Following pupil dilation with 1% (w/v) tropicamide and 2.5% (w/v) phenylephrine, mice were anaesthetised using a ketamine/medetomidine mixture and then imaged in the FA mode with sodium fluorescein (100 mg/kg, i.p.) used as a contrast agent to visualise retinal blood vessels. A-scan images (30° field of view, 1536 x 1536 pixels, average of 100 ART frames) were captured at regular intervals (30 s) between 2 and 8.5 minutes post fluorescein injection, using the Heidelberg Eye Explorer software (version 1.7.1.0). Regions of interest (CNV lesion site(s), inner vasculature outside the CNV(s), microvessels) were selected and quantified in aligned images using ImageJ. To account for inter-individual variability, the signal intensity at each time point was normalised to the starting point and expressed as % value at 2 minutes.

## Expression and Purification of recombinant mouse DEVD-S31-IL-36b and caspase-3

A modified form of murine IL-36b containing a caspase-3-processing motif (DEVD) inserted N-terminal to known processing sites in IL-36b at Ser-31 was generated by cloning the DEVD-modified mouse IL-36b coding sequence in frame with the poly-histidine tag sequence in the bacterial expression vector pET45b. DEVD-S31- IL-36b was expressed by addition of 600 uM IPTG to exponentially growing cultures of endotoxin-free E. coli (ClearColi) bacteria followed by incubation for 3 h at 37°C. Bacteria were lysed by sonication and poly-histidine tagged proteins were captured using nickel-NTA agarose (Amintra, Expedeon Ltd), followed by elution into PBS, pH 7.2, in the presence of 100 mM imidazole. Recombinant poly-histidine-tagged caspase-3 was expressed and purified similarly. Bacterial cell lysates and purified proteins were visualised on 12% SDS-PAGE elecrophoresis gel stained with Coomassie Brilliant Blue.

## IL-36$\beta$ in vivo viability assay

TUNEL staining was used to determine if intravitreal injection of mIL-36$\beta$ resulted in photoreceptor apoptosis. C57BL/6J mice were injected intravitreally with vehicle or mIL-36$\beta$. Mice were sacrificed three days post injection and eyes were paraffin embedded and sectioned. After rehydration, TUNEL staining was performed using the in situ cell death detection kit, TMR red (Sigma), per manufacturer's instructions, and sections were counterstained with DAPI. Sections were imaged by confocal microscopy.

**IL-36 Statistical Analysis**

Data are presented as mean plus or minus standard error of the mean. Statistical analysis of FFA permeability profiles given by two-way ANOVA of matched variables. Statistical analysis of liCNV volume given by one-way ANOVA with multiple comparisons. Statistical comparisons for in vitro experiments were performed comparing treatment to no-treatment control using Student t-tests or ANOVA as indicated.

**IL-18 clinical cohort**

To examine changes in the inflammatory profile within the eye of AMD patients undergoing anti-VEGF treatment, a hospital cohort of treatment naive AMD patients were recruited from the Royal Victoria Eye and Ear hospital in Dublin. A total 46 participants were enrolled in the study, of whom, 33 were recently diagnosed with neovascular AMD and 13 "Healthy" controls having cataract surgery. Inclusion criteria for the study depended on patients being newly diagnosed with exudative AMD requiring treatment with intra-vitreal anti-VEGF; no previous intra-ocular treatment in the study eye; no ocular co-morbidities and patient's ability and willingness to consent to treatment and participation in the study. Informed written consent was obtained at the beginning of treatment course.

**Aqueous sampling and anti-VEGF treatment**

Sample collection and treatment was carried out in a dedicated room under sterile conditions. Three drops of 1% proxymethacaine was administered and allowed to act for ten minutes. One drop of 1% tropicamide was instilled in relevant eye to dilate pupil. With patient lying semi-supine anti-sepsis was carried out using bethadine drops in the eye as well as the peri-orbital area. A single use spring speculum was placed and a drop of 1% proxymethacaine was instilled over the injection site. Aqueous sampling was carried out using a 30-gauge needle attached to a 1ml syringe with approach from the temporal side just medial to limbus and in the horizontal plane. A sample of 0.05ml to 0.1ml of aqueous humor was obtained and transferred immediately to a -80°C freezer for storage. For intra-vitreal injection of anti-VEGF, patients were asked to look in the opposite direction to the injection site. A 28-gauge needle was inserted pointing to the posterior pole of the eye and 125mg/0.05ml of bevacizumab (Avastin®) was injected.

**Best corrected visual acuity (BCVA)**

Visual acuity was examined on all patients at recruitment using a Snellen chart via projector (Nidec) at a distance of 6 meters with the patient seated. All visual acuities were obtained at the same centre and by the same member of staff. The best corrected visual acuity (BCVA) was obtained for each eye alone, with fellow eye fully occluded while using the patient's most up to date correction (spectacles or contact lenses) and then with the addition of a pin-hole. Snellen visual acuities were recorded and then converted to Visual Acuity Score (VAS) (Table 1). Visual acuities were repeated at the 12-week follow up, before the 3rd anti-VEGF treatment.

**Table 1.** Visual Acuity score conversion table

| Line Number | LogMAR | Visual Acuity Score | Snellen Feet 20/ | Metric Meter 6/ | Decimal |
|---|---|---|---|---|---|
| -3 | 0.30 | 100 | 10 | 3.00 | 2.00 |
| -2 | 0.20 | 100 | 12.5 | 3.80 | 1.60 |
| -1 | 0.10 | 100 | 16 | 4.80 | 1.25 |
| 0 | 0.00 | 100 | 20 | 6.00 | 1.00 |
| 1 | -0.10 | 95 | 25 | 7.50 | 0.80 |
| 2 | -0.20 | 90 | 32 | 9.60 | 0.63 |
| 3 | -0.30 | 85 | 40 | 12.00 | 0.50 |
| 4 | -0.40 | 75 | 50 | 15.00 | 0.40 |
| 5 | -0.50 | 65 | 63 | 18.90 | 0.32 |
| 6 | -0.60 | 60 | 80 | 24.00 | 0.25 |
| 7 | -0.70 | 50 | 100 | 30.00 | 0.20 |
| 8 | -0.80 | 40 | 125 | 37.50 | 0.16 |
| 9 | -0.90 | 30 | 160 | 48.00 | 0.13 |
| 10 | -1.00 | 20 | 200 | 60.00 | 0.10 |

(continued)

| Line Number | LogMAR | Visual Acuity Score | Snellen Feet 20/ | Metric Meter 6/ | Decimal |
|---|---|---|---|---|---|
| 11 | -1.10 | 17 | 250 | 75.00 | 0.08 |
| 12 | -1.20 | 15 | 320 | 96.00 | 0.06 |
| 13 | -1.30 | 10 | 400 | 120.00 | 0.05 |
| 16 | -1.60 | 5 | 800 | 240.00 | 0.03 |
| 20 | -2.00 | 0 | 2000 | 600.00 | 0.01 |

**Ocular coherence tomography (OCT)**

OCT was conducted on all eyes at recruitment and at the 12-week follow up assessment using the Cirrus HD-OCT 5000 (Zeiss). Central macular thickness (CMT) was recorded in micro-meters ($\mu$m).

**Objective Impression**

A final determination of response to treatment was made following observation of the anatomical structure of the eye along with OCT and BCVA measurements by the Consultant Ophthalmic Surgeon with extensive experience in treating patients with macular conditions and this decision was recorded as the Objective Impression.

**Buffer compositions**

Radioimmunoprecipitation Assay Buffer (RIPA): PBS, 1% (w/v) NP-40, 0.5% (w/v) sodium deoxycholate, 0.1% (w/v) sodium dodecyl sulphate (SDS).
Transfer Buffer: 25mM Tris, 192mM Glycine, 20% (v/v) Methanol.
Running Buffer: 25mM Tris, 192mM Glycine, 0.1% (w/v) SDS.
Tris-buffered Saline (TBS): 25mM Tris, 137Mm NaCl, 2.7mM KCI (pH to 7.4)
TBS-Tween (TBS-T): 50mM Tris, 137mM NaCl, 2.7mM KCI, 0.05% (v/v) Tween™20.

**HRMEC cell culture**

Primary human retinal microvascular endothelial cells (HRMECs) (Angio-proteomie) were cultured in Endothelial Growth media MV 2 Kit (Promocell) comprised of Basal Medium MV 2 supplemented with 5% Fetal Calf Serum (FCS; Sigma), 1% Penicillin/Streptomycin (P/S; Sigma) and a supplement pack containing 5ng/ml Epidermal Growth Factor (recombinant human), 10ng/ml Basic Fibroblast Growth Factor (recombinant human), 20ng/ml Insulin-like Growth Factor (long R3 IGF), 0.5ng/ml Vascular Endothelial Growth Factor 165 (recombinant human), 1$\mu$g/ml Ascorbic Acid and 0.2$\mu$g/ml Hydrocortisone.
HRMECs were grown on flasks and plates coated with Fibronectin at 1$\mu$g/cm$^2$ (Sigma) diluted in Dulbecco's Phosphate Buffer Saline (DPBS; Sigma). Cells were maintained as monolayers with media changes every 2 days and passaged once a week. To passage cells, culture media was removed, and cells were washed twice with 5ml of DPBS, the second wash was left on the cells for 5 minutes before removing. After the DPBS was removed cells were incubated with 2mL of 0.25% Trypsin-EDTA (Sigma) for 30 seconds before being aspirated off. The flask was placed into an incubator for 1 minute at 37°C to allow adherent cells to dissociate. After incubation the cells were suspended in 4ml of EGM-MV2 culture media. Cells were split 1:4 or seeded at a density of 1-2x10$^5$ cells/ml. Cells were maintained at 37°C with 5% $CO_2$.

**Primary Mouse Brain microvascular endothelial cell isolation**

The following protocol for isolation of primary brain microvascular endothelial cells (BMVECs) was adapted from the methods of Abbott et al (Abbott, N. J., Hughes, C. C., Revest, P. A., & Greenwood, J. (1992). Development and characterisation of a rat brain capillary endothelial culture: towards an in vitro blood-brain barrier. J Cell Sci, 103 ( Pt 1), 23-37) and Assmann et al (Assmann, J. C., Muller, K., Wenzel, J., Walther, T., Brands, J., Thornton, P., ... Schwaninger, M. (2017). Isolation and Cultivation of Primary Brain Endothelial Cells from Adult Mice. Bio Protoc, 7(10). doi:10.21769/Bio-Protoc.2294). The following buffers were prepared prior to isolation:

Working Buffer: Ca2+/Mg2+ free HBSS, 10mM HEPES, 0.5% (w/v) BSA, 1% P/S.

Complete digestion medium: HBSS, 1mg/ml collagenase/dispase, 10mM HEPES, 20u/ml DNase I, 0.147μg/ml TLCK, 1% P/S.

The following instruments were disinfected with 70% EtOH and irradiated with UVC light prior to isolation:

- 1 large surgical scissors
- 1 mini dissection scissors
- 1 fine point straight forceps
- 1 fine point curved forceps
- 1 Scalpel with holder
- 1 dounce tissue grinder
- 1 sheet of Whatman filter paper

Prior to beginning isolation, culture plates were coated with 100μg/ml Collagen IV (Sigma) and 50μg/ml Fibronectin in PBS, plates were incubated at 37°C for 2 hours.

Wildtype (WT) C57BL/6J mice were sacrificed by $CO_2$ asphyxiation followed by cervical dislocation. The head was sprayed with 70% ethanol and removed with large surgical scissors posterior to the ears. The skin was peeled back to expose the skull and excess tissue was removed. Using fine dissection scissors, two horizontal incisions were made at the base of the skull on both sides and along the midline towards the anterior of the skull where it was cut vertically between the eyes. Using forceps, the skull was peeled back to reveal the brain which was gently lifted and placed in ice-cold working buffer. On a sheet of whatman® filter paper in the laminar flow hood, the olfactory bulb and cerebellum were removed using a scalpel. The meninges was removed by rolling the brain on the filter paper twice using curved forceps and cut in half using a scalpel along the midline. The brain sections were placed into a dounce tissue grinder with 5ml of working buffer and homogenize until a smooth consistency. The homogenate was transferred to a 50ml tube, the tissue grinder was rinsed with working buffer and added to the homogenate before centrifuging at 600g for 5 mins at 4°C. The supernatant was removed, and the pellet was resuspended in 10ml of 22% (w/v) BSA in PBS and centrifuged at1000g for 20 mins at 4°C. This spin produced a thick myelin plug which was carefully recovered and resuspended in again in 22% BSA to recover more vessels. The remaining supernatant was aspirated off and the pellet was resuspended in complete digestion medium and incubated at 37°C for 1 hour and 15 mins, shaking every 15mins.

Following digestion, the cell suspension was centrifuged at 600g for 5 mins, supernatant was discarded, and pellet resuspended in warm DPBS and spun down for a final time. During this centrifuge step the pre-coated culture plates were removed from the incubator and the coating buffer was aspirated and wells were washed once with DPBS and allow to air dry.

Following centrifugation in PBS, the vessel fragments were resuspended in selection media which is EGM-MV2 medium with all supplements plus 5μg/ml puromycin, before seeding onto culture plates. BMVECs are resistant to toxic levels of puromycin due to their high expression of P-glycoprotein (Pgp) an integral membrane bound efflux protein (Tsai, C. E., Daood, M. J., Lane, R. H., Hansen, T. W., Gruetzmacher, E. M., & Watchko, J. F. (2002). P-glycoprotein expression in mouse brain increases with maturation. Biol Neonate, 81(1), 58-64. doi:10.1159/000047185). The cells were first incubated in this medium for 24 hours, then medium was aspirated to remove dead cells, and wells were washed with warm DPBS before a second incubation with puromycin supplemented EGM-MV2 medium for 24 hours. On day 3 post seeding the puromycin supplemented medium was removed and cells maintained in EGM-MV2 growth media for 5 days until confluent.

## Isolating and culturing mesenchymal cells from bone marrow

The femurs from mice were isolated and, using a 20ml syringe, the bones were flushed with DMEM into a cell culture dish. The flushings were centrifuged for 5 minutes at 1000rpm. The supernatant was poured off and resuspended in 1ml of 15% FBS and aMEM media, and filtered through a 70um nylon mesh into a 50ml captube. A further 5ml of 15% FBS and aMEM were added through the mesh tube into the same 50ml cap tube, and plated in a TC coated dish and cultured in an incubator for 5 days. On Day 5, the dish was washed with PBS 3 times (using PBS with no magnesium or calcium),trypsinized and the trypsin pipetted onto the plate to break up the colonies. 10mls of 10%FBS and DMEM were added and the cells colected in a 50ml cap tube and this process was repeated once more, centrifuged for 5mins at 1 000rpm, supernatant poured off, cells resuspended in 1ml of DMEM and 20% FBS, and plated in a T75 and fed every second day until cells were confluent. When cells were confluent, they were split 1 in 3.

## Cell stimulations

Confluent HRMECs were stimulated with 50 or 100 ng/ml of recombinant human IL-18 (SB-485232; GSK); 10 ng/ml of human IL-1α (R&D systems) or 50 ng/ml of human VEGF$_{165}$ (Peprotech) diluted in complete growth medium, with complete growth media added to wells alone for 'no treatment'. Primary BMVECs were treated with 100 ng/ml of recombinant mouse IL-18 (SB-528775; GSK), mouse IL-1α at 10 ng/ml (Peprotech) or 50 ng/ml of mouse VEGF$_{164}$ (R&D Systems) diluted in complete growth medium with growth media added to wells alone for 'no treatment'.

**MTS cell viability assay**

Cell viability was assessed using the CellTiter 96 AQueous One Solution cell proliferation MTS assay (Promega) as per the manufacturer's instructions. Cell monolayers were treated for times indicated where treatment supplemented media was then removed and incubated with 20μl of CellTiter 96 AQueous One Solution in 100μl of complete growth medium in a humidified incubator at 37°C with 5% CO$_2$ for 1-4 hours. The absorbance of each well was measured at 490 nm using a SynergyMX plate reader (Biotek, Gen5™).

**Trans-endothelial electrical resistance (TEER) measurement**

Trans-endothelial electrical resistance of endothelial monolayers grown on 0.4μM transwell inserts (VWR-Corning) were measured using the EVOM2 epithelial volt/ohm meter, with STX2 hand-held "Chopstick" electrodes (World Precision Instruments). The STX2 electrodes were sterilized in 70% ethanol for 30 secs and washed in DPBS to remove excess alcohol. The electrodes were calibrated in culture media before taking the first measurement. To measure the endothelial barrier resistance the EVOM2 function was set to Ohms and a 'blank' measurement was taken of a transwell insert with no cells in culture media. The electrode was positioned perpendicular to the transwell with the longer electrode in the external media touching the bottom of the outer well and the shorter electrode in the internal media inside the transwell insert. The electrode was held constant in this position to achieve consistent readings between wells. When changing between treatment wells the electrode was washed and calibrated again to avoid cross-contamination. The true tissue resistance was calculated using the following equation:

$$\Omega_{\text{True Tissue}} = \Omega_{\text{Total}} - \Omega_{\text{Blank}}$$

where:

$\Omega_{\text{Total}}$ is the resistance of the treatment well
$\Omega_{\text{Blank}}$ is the resistance of the blank well
$\Omega_{\text{True Tissue}}$ is the resistance across the endothelial cell monolayer

The unit area resistance was calculated by multiplying the $\Omega_{\text{True Tissue}}$ by the surface area of the membrane and expressed as $\Omega cm^2$.

**Permeability Assay**

Permeability flux assays were performed on HRMECs and mouse primary BMVECs. For HRMECs cells were seeded at a density of 2 x 10$^5$ cells/mL onto 6.5mm Transwell® inserts with 0.4μm pore size (Corning) pre-coated with Fibronectin. Cells were maintained for a minimum of 5 days before start of treatments, with medium changed in the apical and basolateral chambers every 2 days. For primary BMVECs, transwells were coated with Collagen IV and Fibronectin as described. Following isolation, 200μl of vessel fragments were seeded onto transwells in EGM-MV2 growth medium supplemented with puromycin. This medium was replaced with fresh EGM-MV2 and puromycin after 24 hours and then maintained in EGM-MV2 alone following a further 24 hours. Cells were left to reach confluency for a minimum of 5 days before beginning treatments.

Experimental wells were treated with either IL-18 (100ng/ml), IL-1a (10ng/ml) or VEGF (50ng/ml) for 24 hours before assay, or 48 hours in pre-treatment wells. Prior to starting assay, fresh EGM-MV2 medium was placed in the basolateral chamber and the apical chamber medium was replaced with 1mg/ml of FITC-isothiocyanate Dextran (4 or 70 kDa) (Sigma) diluted in EGM-MV2 medium. Immediately following this, for a baseline 0 timepoint, a 100μl aliquot was taken from the basolateral chamber of treatment and control wells and transferred to a 96-well plate (Corning), wells were replenished with 100μl of fresh EGM-MV2 medium and further samples were taken at 20 minute intervals for 2 hours.

The amount of FITC-dextran that passed through the endothelial monolayer into the basolateral chamber was determined using a spectrofluorometer (Optima Scientific) at an excitation wavelength of 485 nm and an emission wavelength of 520 nm. The relative fluorescent units were converted to ng/ml by use of a FITC-dextran standard curve (ranging from 1 mg/ml to 0.001μg/ml) and the apparent permeability coefficient (P$_{app}$) was calculated as follows:

$$P_{app} \, (\text{cm/s}) = dQ/dT / (A \times C_o)$$

where:

dQ/dT (mg/s) is the rate of appearance of FITC-dextran in the basolateral chamber after application, dQ/dT is the slope m(y=mx+c) calculated by plotting the cumulative amount (Q) versus time (T).

A ($cm^2$) is the effective surface area of the insert size.

$C_o$ (mg/ml) is the initial FITC-dextran concentration in the apical chamber.

## Wound healing (Scratch) assay

Cells were seeded on to a 12-well plate and grown until they reached confluency. 24 hours prior to the scratch, culture medium was replaced with 0.5% Serum media without supplements for 24 hours to induce cell quiescence. Cell monolayers were scratched once down the centre of the well using a p200 pipette tip and washed once with warmed DPBS to remove debris then replaced with 0.5% serum supplemented media with experimental treatments. Images were acquired using an Olympus IX51 inverted microscope with Cell^A imaging software (Olympus). Images were taken at baseline immediately following scratch and at suitable timepoints for up to 24 (HRMECs) or 48 hours (BMVECs). The area within the scratch was quantified using ImageJ software, and each timepoint was normalised to its baseline and expressed as % closure.

## Protein Isolation from cells

Cell culture supernatant was aspirated, and wells were washed once with ice cold DPBS. On ice, cells were lysed in RIPA buffer (PBS, 1% NP40, 0.5% sodium deoxycholate and 0.1% SDS) containing a 1:100 dilution of a protease and phosphatase inhibitor cocktail (Sigma). Wells were scraped with a p200 pipette tip and lysates were transferred to an Eppendorf tube and centrifuged for 15 mins at 14,000g to remove cell debris and immediately stored at -20°C.

## Determination of protein concentration

Protein was quantified using the Pierce™ Bicinchoninic acid assay kit (BCA assay) (Thermo Fisher Scientific) as per the manufacturer's instructions. Protein standards were made by serial dilution of bovine serum albumin (BSA) in the range of 0 - 2,000 µg/ml. Samples were added to wells of a 96 well plate in duplicate. Working BCA reagent was prepared by mixing 50 parts of BCA reagent A to 1-part BCA reagent B. This working reagent was added to wells and plate was incubated at 37°C for 30 minutes. Absorbance was measured on a Synergy™Mx microplate reader (BioTek) at 562nm.

## SDS-PAGE Gel Preparation

SDS-PAGE resolving and stacking gels were handmade. Gels were made and run using the mini-PROTEAN® tetra hand cast system (Bio-Rad). Casting plates consisting of a short and 1.5mm spacer plate were assembled and held in place by clamps in a casting frame. Resolving gel was poured into the plates leaving enough space for the stacking gel, corresponding to the length of the comb plus 1cm. An overlay solution of water saturated n-butanol was applied to prevent drying out of the gel as it set. Following polymerisation, the n-butanol was removed, and the top of the resolving gel was washed thoroughly with distilled water before addition of the stacking gel and comb.

Once set, gels were taken out of the casting frame, the comb was removed, and gels were inserted into a vertical electrophoresis module with short plates facing the inner chamber. The module was placed into a gel tank and the inner and outer buffer chambers were filled with 1X Running buffer. Gels were loaded with protein samples and standards and initially run at 100 volts to allow samples to stack then increased to 150 volts for separation in the resolving gel.

## Sample Preparation

5µl of 5X sample buffer (250nM Tris pH 6.8, 10% w/v SDS, 50% Glycerol, 0.5% Bromophenol Blue, 500mM DTT) was added to 20ul of protein samples and boiled at 95°C for 5 mins and allowed to cool. An equal volume of sample was loaded into each well alongside 8µl of PageRuler™ Plus prestained ladder (Thermo-Fisher).

## Transfer of proteins

Proteins were transferred onto a polyvinylidene difluoride (PVDF) membrane (Millipore) using the mini Trans-Blot® cell system (Bio-Rad). Following electrophoresis, the short casting plate was carefully separated from the gel and the stacking gel was discarded. PVDF, activated in 100% Methanol, was placed on top of the gel and the gel/PVDF was sandwiched between filter paper soaked in 1X Transfers buffer and placed inside the transfer cassette. A cooling unit was added to the gel tank to prevent the buffer overheating and the tank was filled with 1X transfer buffer and run at 100 volts for 1 hour.

### Immuno-blotting

Following transfer, membranes were blocked by incubating in 5% non-fat dried milk (Marvel) in TBS-T for 1 hour at room temperature whilst shaking. Membranes were incubated with primary antibody diluted in either 5% Marvel or BSA, depending on antibody specification, overnight at 4°C with gentle agitation. Primary antibody was recovered and stored at -20. Membranes were washed three times in TBS-T for 5 minutes and membranes were incubated with secondary antibody, diluted in 5% Marvel, for 1 hour at room temperature. Secondary antibody was removed and discarded, and excess secondary was removed from the membranes by three 5-minute washes in TBS-T.

Membranes were removed from TBS-T and allowed to dry for 20 seconds before marking proteins standards using a WesternBright™ ChemiPen™ (Advansta). Proteins were detected using an in-house enhanced chemiluminescence (ECL) reagent or the WesternBright™ ECL HRP substrate kit (Advansta). In both cases, equal volumes of substrate A and B were mixed and immediately pipetted over membrane following marking of protein standards. The membrane was sandwiched between to sheets of acetate in a film cassette, and in the dark room, a sheet of X-Ray film (FujiFilm) was placed over the membrane and sealed in the cassette. Exposure time varied with each antibody. Following exposure, the X-Ray film was removed and submerged in a bath of developer solution until protein signal was observed. The film was washed in a bath of water before being placed in a bath of Fixer solution and left to incubate for 30 seconds with gentle agitation. Excess fixer was washed away, and film was left to dry. Alternatively, after incubating with ECL reagent, membranes were placed in the LAS-3000 Imager (Fujifilm) using the LAS-3000 software to focus and control exposure times. Densitometry on images was carried out using ImageJ software to calculate the band intensity for each treatment sample and normalising these to the band intensity of the loading control protein β-Actin.

### Plasma collection

Whole blood was collected in EDTA tubes and centrifuged at 10,000g for 5 minutes. Plasma was carefully transferred into 1ml aliquots and stored at -80°C until assessment.

### IL-18 ELISA

Plasma levels of IL-18 were measured by sandwich ELISA. A 96 well plate was coated with 100$\mu$l of 0.5$\mu$g/ml anti-human IL-18 capture antibody (R&D Systems) diluted in 50mM sodium carbonate-bicarbonate buffer (pH 9.4) (Thermo Scientific) and incubated overnight at 4°C. Plate was washed in TBS with 0.1% Tween X3 and blocked using SuperBlock™ TBS for 1 hour at room temperature. Plate was washed X3 and 50$\mu$l of plasma was added in triplicate to the plate. Samples were left to incubate for 2 hours at room temperature while shaking. Plate was incubated with 100$\mu$l of 100ng/ml biotinylated anti-human IL-18 detection antibody (R&D Systems), diluted in reagent diluent, and incubated for 1 hour at room temperature while shaking. Excess antibody was washed away X3 in TBS-T and plate was incubated with Streptavidin-HRP reporter tag (Thermo Scientific) for 20 minutes whilst shaking. Plate was protected from light from this step onwards. Plate was washed X3 before the addition of 3,3',5,5; -Tetramethylbenzidine (TMB) (Sigma) and left at room temperature to allow oxidisation of the solution by HRP and form a blue precipitate. This reaction was stopped using a STOP solution of 1:12 parts HCl to water. The absorbance was read on a Synergy™Mx microplate reader (BioTek) at 450nm.

### IL-18 Binding Protein (IL-18BP) ELISA

The concentration of IL-18BP was in plasma was determined using a DuoSet® ELISA kit (R&D Systems). Capture antibody was diluted to a working concentration of 2$\mu$g/ml in DPBS and a clean ELISA plate (Greiner Bio-One) was coated with 50$\mu$l of this solution, sealed, and allowed to incubate overnight at room temperature. Plate was washed X3 in PBS-T (0.05%) wash buffer and 200$\mu$l of reagent diluent, 1% BSA in PBS, was added to block wells. Plate was blocked for 1 hour at room temperature with gentle agitation before being washed, as before, and 50$\mu$l of plasma was added. Plasma samples were added in triplicate and allowed to incubate for 2 hours at room temperature while shaking. Plasma was removed and plate washed before the addition of detection antibody, diluted to a working concentration of 25ng/ml in reagent diluent and left to incubate for 1 hour at room temperature while shaking. Following this, the plate was washed and the Streptavidin-HRP, included in the kit, was diluted 1:20 in reagent diluent and 50$\mu$l added to each well. The plate was protected from light from this stage forward. The Strep-HRP solution was washed off, and 50$\mu$l of TMB solution was added and left to incubate until a blue colour was observed, after sufficient time an equal amount of STOP solution was added to each well to stop the reaction and the absorbance was read at 450nm.

**Free IL-18 Calculation**

The concentration of free IL-18 which is the fraction of cytokine not bound to its inhibitor IL-18BP, was calculated using the law of mass action (Migliorini et al., 2010). Following measurement of total IL-18 and IL-18BP concentrations by ELISA, the calculation was applied based on knowing the molecular weight of IL-18 (18.4 kDa) and IL-18BP (17.6 kDa), the 1:1 stoichiometry of the IL-18/IL-18BP complex and the dissociation constant ($K_d$) of 0.4nM.

The law of mass action:

was applied as follows:

$$K_d = \frac{[Ligand] \cdot [Receptor]}{[Ligand \cdot Receptor]}$$

where:

$$x = \frac{-b + \sqrt{b^2 - 4c}}{2}$$

$x = [\text{IL-18}]_{free}$

$$b = [\text{IL-18BP}] - [\text{IL-18}] + K_d$$

$$c = -K_d \times [\text{IL-18}]$$

**RNA Isolation**

RNA from all cell lines was isolated using the E.N.Z.A.® Total RNA kit I (Omega BIO-TEK) according to the manufacturer's instructions. Following treatments, culture medium was removed, and wells were washed once with DPBS. Cells were lysed by the addition of 350$\mu$l of TRK Lysis Buffer directly onto the cell monolayer and homogenised by repeated pipetting up and down. An equal volume (350$\mu$l) of 70% ethanol was added to the homogenate and mixed by pipetting. 700$\mu$l of sample was transferred to a HiBind® RNA Mini Column inserted into a collection tube and samples were centrifuged at 10,000g for 1 minute, the filtrate in the collection tube was discarded. The HiBind® Mini column was washed once with 500$\mu$l of Wash Buffer I, centrifuged at 10,000g for 30 seconds and filtrate discarded, then twice with RNA Wash Buffer II supplemented with 200ml of 100% Ethanol (EtOH). Following these washes the empty HiBind Mini column was spun at maximum speed for 2 minutes to remove any remaining ethanol or trace contaminants. The column was transferred to a clean 1.5ml Eppendorf tube and 41$\mu$l of Nuclease-free water was applied directly onto the filter membrane and samples were centrifuged at maximum speed for 2 minutes to elute RNA. RNA yield (ng/$\mu$l) and quality ($A_{260}/A_{280}$) was assessed using a NanoDrop™ 1000 spectorphotometer (Thermo Scientific).

**cDNA Synthesis**

All RNA samples were diluted to 50ng/$\mu$l before cDNA synthesis. Reverse transcription of RNA was done using 0.25$\mu$l M-MLV Reverse Transcriptase (10units/$\mu$l) (Promega) in a master-mix consisting of 2$\mu$l 5X M-MLV buffer (Promega), 2$\mu$l dNTP's (10mM)(New England Biolabs), 0.5$\mu$l Random Hexamer (2$\mu$M) (IDT) and 0.25$\mu$l RNase out (40units/$\mu$l)(Invitrogen). Equal volume of RNA to master-mix was added to RNAase/DNAse free pcr tube and run on a Veriti 96-well thermo cycler (Applied Biosystems) and run at 20°C for 10 minutes, 42°C for 40 minutes and 95°C for 3 minutes. Following synthesis, cDNA was diluted 1 in 2 (25ng) or 1 in 10 (5ng) in RNAse free $H_2O$ and stored at 4°C.

**Real-Time quantitative PCR (qPCR)**

All RT-PCR assays were carried out using the SensiFAST SYBR Hi-ROX Kit (Bioline) with primers designed using Primer-BLAST (NCBI). For each target a master-mix comprising of 4$\mu$l SYBR Green mix, 0.5$\mu$l of Forward primer (4$\mu$M) and 0.5$\mu$l of Reverse primer (4$\mu$M) along and 3$\mu$l of RNAse free $H_2O$ was prepared and mixed thoroughly by

vortexing. 8μl of master-mix was added to each well of a 0.1mL MicroAMP® Fast 96-well Reaction Plate (Applied Biosystems), along with 2μl of cDNA and sealed using Optical adhesive film (Applied Biosystems) and spun down for 30 seconds in a centrifuge fitted with microplate rotor (Eppendorf) before running. Plate were run using a QuantStudio 3 real-time PCR System (Applied Biosystems) or the StepOnePlus Real-Time PCR System (Applied Biosystems), with run conditions set as: 50°C for 2 mins, 95°C for 10 minutes, 40 cycles of 95°C for 15 seconds then 60°C for 1 minute. To assess the quality of Primers a melt-curve stage was added: 95°C for 1 second, 60°C for 20 seconds, 95°C for 1 second. Data were analysed using the QuantStudio™ Design & Analysis software (Applied Biosystems) and using the comparative $C_t$ ($\Delta\Delta C_t$) method where test samples were normalised to endogenous control (β-Actin) followed by normalising the $\Delta$Ct of test samples to sample control (untreated sample), then calculating $2^{-\Delta\Delta Ct}$ to find the relative fold change of test samples over sample control.

**IL-18 Statistical analysis**

Analysis of aqueous cytokines was performed using non-parametric Kruskal-wallis test with Dunn's multiple comparison for comparison of independent groups of 3 or more. Mann-whitney t-test was used for 2 independent groups. Correlation analysis of cytokines with $\Delta$BCVA or $\Delta$OCT was done using Spearman's rank correlation. Analysis of change in cytokine levels between baseline and week 12 between those who improved or deteriorated was carried out using Two-way ANOVA with samples matched between timepoints, with Sidak;s multiple comparison test between Improved/Deteriorated groups and Tukey's multiple comparison test between timepoints.

The invention will now be described with reference to the following non-limiting examples:

**Example 1**

**Loss of IL-36 Receptor Antagonist results in microvascular remodelling in the CNS**

To examine the effects of unchecked IL-36 signalling on microvascular integrity of the central nervous system (CNS) in vivo, the retinal vascular network was examined in 12 month old mice lacking IL-36 Receptor Antagonist (IL-36rn-/-) for structural changes. The retina is part of the CNS and retinal blood vessels are functionally analogous to the cerebral blood vessels. Using isolectin staining to highlight the vasculature in retinal flatmounts, it was evident that there were alterations to the retinal vascular structure in the IL-36rn-/- mice compared to age-matched wild-type controls, with a greater density of vessels observed in the IL-36rn-/- mice (Figure 1A).

To quantify whether these observed differences were significant, REAVER analysis was employed to measure total length of vessels, number of branchpoints and vascular tortuosity in representative flattened Z-stack images from wild-type and IL-36rn-/- mice (Figure 1B).

Maximum fluorescent intensity (MFI) of these representational images was also measured in order to ascertain vascular density. IL-36rn-/- mice had significantly increased retinal vascular length, increased incidence of vascular branching points and an increase in overall vascular density compared to wild-type animals (Figures 1C-E).

There was no evidence for an increase in tortuosity in the IL-36rn-/- mice (Figure 1F). These data would suggest that excess IL-36 signalling can lead to the structural remodelling of the retinal microvasculature.

**Example 2**

**Microvascular endothelial cells of the CNS are directly activated by IL-36 cytokines**

The vascular network analysis of the IL-36rn-/- mice demonstrated that IL-36 signalling affected microvessel geometry. However, IL-36 could be acting in a direct or indirect manner on the endothelial cells. A pro-angiogenic role for IL-36 via indirect mechanisms in non-CNS related disease has been suggested.

The publicly available endothelial translatome database (http://www.rehmanlab.org/ribo) was utilised to establish whether endothelial cells in the CNS actively express IL-36R (ilr1l2) and family cytokine receptors. This resource provides information on genes undergoing active translation under baseline conditions and post inflammatory (LPS) challenge in vivo.

It was found that brain endothelial cells actively express IL-36R (Il1rl2), IL-18R (il18r1), IL-18R accessory protein (il18rap), IL-1R accessory protein (Il1rap), and IL-1R (il1r1) basally with increased expression during inflammation of IL-36R, IL-18Rap and IL-1R (Figure 2A).

Next, expression levels were examined in primary human retinal microvascular endothelial cells (HRMECs). Real-time qPCR was performed on RNA isolated from confluent HRMEC monolayers. Expression levels of IL-18R, IL-18RAP, IL-1RAP and IL-36R were compared using β-actin as the housekeeping gene and are presented relative to IL-18R (as the least abundant receptor). Both IL-36R mRNA, and the accessory protein IL-1 RAcP, required for IL-36 signalling, are endogenously expressed at prominent and comparable levels basally in HRMECs (Figure 2B).

Localisation of IL-36R was next assayed in HRMEC by confocal microscopy. HRMEC were grown to confluency and stained for IL-36R and endothelial-specific plasma membrane protein VE-cadherin. IL-36R was found at the cell membrane, where it co-localizes with the endothelial adherens junction component VE-cadherin (Figure 2C, closed white arrows).

It was also found in the nucleus, indicating that like some other IL-1 family receptors IL-36R contains a nuclear localization signal (Figure 2C, open red arrows). Indeed, analysis of the IL-36R protein amino acid sequence using cNLS Mapper revealed two putative bipartite nuclear localisation signals from residues 501 and 506, with high NLS scores of 6.6 and 5.9 respectively.

It was next examined whether IL-36R is capable of signalling in HRMECs. IL-36R, like other IL-1R family members, activates the transcription factor NFκB and mitogen activated protein kinase (MAPK) p38 in a number of cell types. HRMECs were cultured to confluency, and treated with recombinant human IL-36$\alpha$ or IL-36$\beta$ over a 90 minute timecourse. IL-36$\alpha$ and IL-36$\beta$ treatment resulted in phosphorylation of both NFκB p65 (Ser536) and MAPK p38 (Thr180/Tyr182) within 5 minutes of stimulation with cytokine (Figures 2D & E), confirming that IL-36 signalling pathways are functional in HRMECs.

To identify IL-36- regulated functional effects on endothelial cells in an unbiased manner, RNA sequencing was performed on HRMECs treated with IL-36$\beta$ for 24h and network analysis of the data was undertaken using iDEP and ShinyGO (Figure 2F). Visualisation of the relationship among enriched GO terms using a hierarchical clustering tree identified the top pathways impacted by IL-36 were 'vasculature development' and 'blood vessel morphogenesis', in addition to 'angiogenesis' and related pathways; 'cell proliferation', 'cell migration' and 'cell adhesion' among others.

### Example 3

**IL-36 cytokines directly facilitate microvascular remodelling in vitro**

The ability to form vessels or tubes is essential for the process of vascular remodelling and reorganisation, newly formed vessels can then mature or undergo regression. The increased branch points in the IL36rn mice and the network analysis of gene expression changes downstream of IL-36 stimulation of HRMECs implied a role for IL-36 cytokines in microvascular remodelling. A Matrigel-dependent tube formation assay was utilised to determine the effect of IL-36 cytokines on this process in HRMECs that have the capacity to develop mature tubes and a visible network 4 hours post seeding. Figure 3A depicts representations of network complexity for IL-36 cytokine treatments.

IL-36$\alpha$ demonstrated a potential ability to increase the length of the tubes but not the complexity of the network, indicated by number of branching points, when compared to the unstimulated control (Figure 3B).

IL-36$\beta$ significantly increased the length of tubes when compared to the unstimulated control (left panel), and is also capable of enhancing network complexity as indicated by a significant increase in branching points (middle panel) and number of tubes (right panel) (Figure 3C). VEGF was used as a control in all cases.

Vessel formation and vessel remodelling are phases in the multi-step process of angiogenesis that also involves proliferation and migration of endothelial cells. To determine whether IL-36 treatment could directly impact proliferation of HRMEC, cells were treated with IL-36$\alpha$ or IL-36$\beta$, both cytokines resulted in an increase in the number of cells after 24 hours (Figure 3D).

In addition, an MTS/PMS assay was performed, which again showed a significant increase in proliferation of cells in response to exposure to IL-36$\alpha$ or IL-36$\beta$ (Figure 3E), although neither cytokine was as effective as VEGF alone.

### Example 4

**IL-36 cytokines directly induce endothelial cell migration**

It was next assessed whether IL-36 cytokines could drive endothelial cell migration. HRMEC were grown to confluency before the monolayer was 'scratched', creating a wound. Cells were then treated with IL-36$\alpha$ or IL-36$\beta$ and images of wound closure were captured periodically to assay migration of cells into the scratch (Figures 4A-F). IL-36$\alpha$ or IL-36$\beta$ treatment applied at the time of the scratch increased the rate of wound closure and cell migration significantly (Figures 4A-F) indicating that IL-36 cytokines are capable of driving endothelial cell migration in vitro.

### Example 5

**IL-36 cytokines directly enhance monolayer integrity and reduce vascular permeability**

One of the most important characteristics of endothelial cells is the ability to form and maintain a stable vascular network. Loss of barrier properties in established vascular networks can lead to disease, and similarly pathology associated with neovascularisation is often due to nascent vessel incapacity to maintain appropriate barrier function. The CNS is an immune privileged tissue, and as such the inner blood retinal barrier (iBRB) allows stringent control over the influx and efflux of material into the retina analogous to the BBB.

These data indicate that IL-36 cytokines possess properties that drive angiogenic processes in vitro, which is usually accompanied with a loss of barrier function. To examine whether IL-36 cytokines can directly affect endothelial monolayer permeability trans-endothelial electrical resistance (TEER) and FITC-dextran trans-endothelial flux assays were performed in HRMECs treated with IL-36α or IL-36β. Assays were performed once cells had reached confluency (Figure 5A).

TEER readings were taken prior to initiation of flux assay and indicated that when compared to baseline values, treatment with either IL-36α or IL-36β both significantly increased TEER compared to untreated control. This indicates that resistance across the HRMEC monolayer barrier is improved by IL-36 cytokines (Figure 5B).

Following either 6 or 24 hour exposure to IL-36α the rate of FITC-dextran migrating across the HRMEC monolayer was notably decreased (Figure 5C).

Accordingly, IL-36α induced significant decreases in apparent permeability (Papp), indicating a direct IL-36a-dependent increase in endothelial monolayer tightness (Figure 5D).

In the case of IL-36β, the decrease in rate of FITC-dextran flux was significant at 24 hours (Figure 5E) with a significant decrease in Papp 24 hr post treatment (Figure 5F).

Together, these data suggest that rather than inducing permeability, IL-36 cytokines in fact enhance HRMEC barrier function in vitro.

## Example 6

### IL-36 cytokines increase expression of tight junction and cadherin junction components

Much of the control of retinal vascular integrity and low rates of fluid phase transcytosis is determined by the endothelial cell barrier complex, which is comprised of tight junctions, gap junctions and adherens junctions controlling influx and efflux of molecules from the circulation to tissue and vice versa. VE-cadherin is a critical regulator of vascular permeability and a component of the adherens junction. Whereas, tight junctions (TJ's), are composed of proteins such as ZO-1, tricellulin, occluding and claudin-5.

Interestingly, analysis of the RNA sequencing dataset indicated that both protocadherin VE-cadherin2 (24 h adjusted p = 0.04) and claudin-5 (24 h adjusted p = 0.001) were two genes highly induced by IL-36β (both in the top 25 most variable genes) (Figure 5G). Furthermore, ShinyGO network analysis pointed to cell-cell adhesion as a pathway significantly regulated by IL-36β (Figure 2F).

Recently RepSox, a TGFβR inhibitor was found to increase barrier integrity through upregulation of TJ protein claudin-5. Gene expression of TJ proteins claudin-5, tricellulin and ZO-1 in response to either IL-36β or RepSox was compared for comparison (Figure 5H), and it was found that IL-36β induced claudin-5 and tricellulin to an equivalent level as RepSox, while ZO-1 transcript appeared unaffected in response to either stimulus. VE-cadherin transcript was next analysed and it was observed that IL-36 cytokines could similarly induce VE-cadherin gene expression in a time dependent manner (Figure 5I).

## Example 7

### Inhibition of protein translation results in loss of IL-36-mediated barrier integrity

It was next examined if the increase in transcript translated into enhanced protein expression levels of VE-cadherin (Figures 6A & B) and TJ proteins ZO-1, tricellulin and occludin (Figures 6C & D) in response to treatment with IL-36α (Figures 6A & C) or IL-36β (Figures 6B & D). Increased expression of VE-cadherin 24 h after IL-36 cytokine treatment was found (Figures 6A & B). Enhanced TJ expression with dose dependent increase in expression of ZO-1, tricellulin and occludin was also observed 24 h after IL-36 cytokine treatment (Figures 6C & D).

Together, these results suggest that the enhancement observed in vitro in barrier function following IL-36 cytokine stimulation may be due in part to upregulation of multiple barrier components by the IL-36 cytokines. To test this it was investigated whether inhibiting protein translation resulted in a loss of IL-36-dependent barrier enhancement. HRMEC grown on transwells were incubated with translation-inhibitor cycloheximide (CHX), and stimulated with IL-36α or IL-36β prior to flux assay. Inhibition of protein translation results in absolute loss of IL-36α- and IL-36β-dependent barrier enhancement (Figures 6E-H) strongly suggesting that IL-36-dependent gene expression and translation of junctional proteins is the primary mechanism by which IL-36 improves monolayer integrity.

**Example 8**

**IL-36β enhances barrier function in the micro-vasculature of the CNS under inflammatory conditions**

The barrier enhancing properties of the IL-36 cytokines in vitro were striking and we wished to examine whether this function translated to the in vivo setting. The retina is supplied with nutrients from two vascular beds, the inner retinal vasculature that contributes to the iBRB, and the outer retinal, fenestrated, choroidal vasculature.

To examine effects of IL-36 on vascular integrity in the CNS in vivo, IL-36β was administered at two doses (0.3 ng/eye or 3 ng/eye) directly into the vitreous of C57BL/6J mice immediately after they received a laser-induced injury in both eyes. Laser-induced injury encourages pathological neovessel growth from the choroidal vessels in the outer retina termed choroidal neovascularisation (CNV). These nascent CNV's have poor barrier properties and are permeable to fluorescein allowing intravital acquisition of dynamic fundus fluorescein angiography (FFA) imaging. FFA was performed at day 5 post IL-36β (Figures 7A-D; E representative images).

IL-36β was chosen over IL-36α as it broadly tended to induce the strongest responses in the in vitro experiments. A striking enhancement of the overall barrier function was observed with 3 ng of IL-36β (Figure 7A). This effect was particularly evident at the site of CNV (Figure 7B). However, in addition, IL-36β-dependent enhanced barrier function was also detected in the inner retinal vasculature, encompassing both the superficial and deep vascular plexus outside the laser injury-affected area (Figure 7C), as well as in microvessels of the deeper vascular plexus (Figure 7D).

These data identify IL-36β as a potential vascular stabilising factor improving barrier integrity at sites of direct injury and at sites of micro-vessel inflammation.

**Example 9**

**IL-36β has therapeutic potential for stabilising pathological vascular permeability**

These data supported a function for IL-36 in enhancing barrier properties and pointed to the potential for IL-36β as a therapeutic regulator of microvascular leakage. However, in such a case, given the data demonstrating IL-36 signalling can induce angiogenic steps in vitro and the evidence for baseline vascular remodelling in vivo, it was necessary to examine the impact of IL-36β on neovascularisation in vivo in a pathological setting. To do this, choroidal neovascular lesion volume was assessed by isolectin (Ib4) staining. Volume analysis indicates that IL-36β does not increase neovascular lesion size, with no significant change in lesion volume observed with either dose (0.3 ng/eye or 3 ng/eye) of IL-36β (Figures 7F, G representative images).

These data demonstrate that IL-36 does not worsen pathological neovascularization. In other non-CNS disease models, IL-36 has been shown to upregulate IL-1a/β expression in immune cells. IL-1a/β can cause pathological infiltration of immune cells, swelling and neuronal cell death. To examine whether IL-36β similarly causes neuronal cell death we injected IL-36β at a range of doses up to 30 ng per eye and performed terminal deoxynucleotidyl transferase dUTP nick end labelling on retinal cross sections to detect DNA fragmentation. Figure 7H demonstrates that in contrast to IL-1, IL-36β does not induce cell death or cause structural abnormality of the neural retinal tissue at concentrations 10 times the dose needed to reduce microvessel permeability.

**Example 10**

**Reciprocal regulation of IL-36 and VEGF signalling**

HRMEC's can respond to both VEGF and IL-36 signalling. These data indicate that there is a reciprocal relationship in their regulation as VEGF treatment reduces IL-36R expression (Figure 8) whereas both IL-36alpha and IL-36beta cytokines can reduce VEGFR2 expression (Figure 9).

Taken as a whole, this IL-36 dataset indicates that IL-36 treatment of neovascular disease might work alone as well as an adjunct to anti-VEGF therapy as we would expect it to (1) reduce the expression of VEGFR2 thereby inhibiting any residual VEGF from signalling in addition to (2) driving VEGF-independent compensatory endothelial cell proliferation in parallel with enhancing cell-cell junctions upon contact thereby stabilising the vascular lesion, resulting in a smaller stable neovascular membrane. Therefore, the combination of anti-VEGF and IL-36 together will be therapeutic.

**Example 11**

**Aqueous IL-18 levels increased significantly over 12 weeks with in-patients with wet AMD given anti-VEGF treatment**

Next, the concentration of endogenous IL-18 within the aqueous of a neovascular clinical cohort was determined and compared to cataract controls. The change in IL-18 concentration was followed during the initial 3-month course of bevacizumab in a hospital cohort analysing the change in IL-18 levels in both responders and non-responders as determined by best corrected visual acuity (BCVA) and optical coherence tomography (OCT) measurements, and determine whether baseline levels of plasma or aqueous IL-18 correlate with overall improvement in these measurements post-treatment. A cohort of treatment naive men and women newly diagnosed with CNV secondary to AMD from the RVEEH and the Beacon Clinic were consented. Prior to administering the first dose of bevacizumab to the treatment naive clinical cohort, blood and aqueous samples were collected to measure the baseline levels of IL-18. IL-18 levels were then grouped based on participant's response to treatment as determined by BCVA and OCT measurements.

From 33 participants, aqueous IL-18 concentration was assessed 3 times over a 12-week period. One baseline sample and two further samples taken at six-week intervals immediately prior to intravitreal bevacizumab injection. We found that there was a significant increase in the levels of aqueous IL-18 between baseline measurements and week 12 ($p$ = 0.01), with the incremental increase in IL-18 levels also significant from week 6 to week 12 ($p$ = 0.02). These data indicate a gradual increase in IL-18 levels over time in line with treatment of anti-VEGF for wet AMD (Figure 10).

**Example 12**

**AMD patients who respond to anti-VEGF therapy have a significant increase in IL-18 in their aqueous humor over 12 weeks**

The aqueous samples were categorised based on a patient's response to bevacizumab into AMD cases that improved or deteriorated over the course of treatment. We found no significant difference in the overall levels between responders and non-responders. However, one striking difference observed between those who responded to treatment and those that did not was that IL-18 concentration in the aqueous rose significantly between baseline and week 12 for those who improved based on all methods of measurement; BCVA ($p$ = 0.01) (Figure 11A), OCT ($p$ = 0.01) (Figure 11B), and objective impression ($p$ = 0.003) (Figure 11C). Improvers as measured by objective impression also saw a significant increase in IL-18 between week 6 and week 12 ($p$ = 0.02). While we did not find a significant increase in IL-18 levels between baseline and week 12 for the BCVA and objective impression in those who did not respond to treatment, we did find a significant difference between IL-18 levels from week 6 to week 12 for non-responders as measured by OCT (P=0.04), indicating a delayed or sub-optimal response to anti-VEGF treatment.

**Example 13**

**Higher levels of aqueous IL-18 correlate with decreased macular oedema**

Correlation between overall change in OCT over the course of treatment and log-transformed IL-18 levels at the 3 sampling stages were assessed. The overall change in OCT yields a negative result, indicating a reduction in central macular thickness (CMT), in patients who improved. There was a moderate inverse correlation between IL-18 at week 6 and ΔOCT (Spearman r = -0.40) (Figure 12) where higher levels of IL-18 correlated with a greater reduction in retinal thickness, a trend that continues at week 12, albeit weaker (Spearman r = -0.25) (Figure 12).

**Example 14**

**Plasma IL-18 correlates with reduced retinal oedema**

There was no significant difference in baseline plasma IL-18 concentration between responders and non-responders based on their BCVA measurement (Figure 13A), with a weak positive correlation between baseline plasma IL-18 concentration and BCVA measurement (Figure 13D). In contrast, it is very clear that those who responded to anti-VEGF treatment with reduced retinal thickness/oedema after the course of treatment, as measured by OCT, had significantly higher levels of plasma IL-18 at baseline than those patients that did not respond to treatment (Figure 13B). Furthermore, IL-18 levels strongly correlated with the overall change in OCT during the course of treatment (Figure 13E), with higher baseline plasma IL-18 correlating with a greater reduction in retinal thickness (Spearman r = -0.47, $*p$ = 0.01).

**Example 15**

**IL-18 can inhibit VEGF secretion in vivo and in vitro**

In the retinas of young animals (3 weeks old), VEGF levels were significantly decreased after treatment with the lower (100 $\mu$g/kg) dose of IL-18, (Figure 14A). The RPE serves many important support functions in order to maintain retinal homeostasis, one of them being production of growth factors, including VEGF. In vitro, the levels of secreted VEGF were found to increase over time in a linear manner, suggesting that VEGF was being constitutively produced by the RPE. Supplementation of the cell culture media with IL-18 (1000 ng/ml) decreased VEGF concentration at (almost) all time points examined (1-72 hours) (Figure 14B) and also significantly slowed down the overall rate of VEGF secretion (Figure 14C).

**Example 16**

**IL-18 reduces expression of VEGF2 specifically in Human retinal endothelial cells (HRMECs)**

HRMECs respond to VEGF through signalling from a variety of VEGF receptors. The main one though to be involved in pathogenic angiogenesis is VEGFR2. IL-18 specifically reduces VEGFR2 on these retinal endothelial cells (Figure 15).

**Example 17**

**IL-18 stimulates increased HRMEC migration across a wound**

Cell migration is an important component in the formation of new blood vessels. During angiogenesis, in response to changes in the surrounding environment, endothelial 'tip' cells use migration to initiate sprouting of new blood vessels, in wound healing this movement of endothelial cells is necessary to repair and maintain vascular integrity. Using the scratch assay as a model of wound healing, we assessed endothelial cell migration in response to IL-18 and IL-1$\alpha$. It was found that IL-18 significantly increased the rate of wound closure when compared to no treatment control, and was comparable to VEGF induced wound closure at 24 hours (Figure 16A). Interestingly, while not significant, it was found that at the 4 and 8 hour timepoint post scratch, VEGF has an increased rate of closure over IL-18. At 8 hours post scratch IL-18 treated cells are comparable with no treatment controls (NT vs IL-18; $\overline{x}_{diff}$ = -0.99, $p$ = 0.99) while the mean difference between VEGF and control wells trends towards significane (NT vs VEGF; $\overline{x}_{diff}$ = -9.35, $p$ = 0.08) with FGF treated cells in between (NT vs FGF; $\overline{x}_{diff}$ = -4.366, $p$ = 0.79). By 24 hours however, the rate of closure for IL-18 treated cells has increased significantly ($\overline{x}_{diff}$ = -12.58, $p$ = 0.002) and is now comparable to VEGF (NT vs VEGF; $\overline{x}_{diff}$ = -12.25 $p$ = 0.01), and both are surpassed by FGF (NT vs FGF; $\overline{x}_{diff}$ = -28.4, $p$ = 0.0001) (Figure 16B). These data suggest that VEGF's influence on endothelial cell migration is strongest in the initial few hour after exposure while IL-18 signaling may be delayed by comparison but can ultimately induce a potent pro-migratory response.

**Example 18**

**IL-18 enhances VEGF induced proliferation in HRMECs**

Using an MTS assay, HRMECs were treated with increasing doses of VEGF, which corresponded to an increase in proliferation up to 5ng/ml were higher doses have minimal advantage (panel 1). This VEGF induced proliferation was increased following a 24 hour pre-treatment with IL-18 at increasing doses. Note IL-18 alone was also able to enhance HRMEC proliferation (Figure 17).

**Example 19**

**IL-18 drives expression of tight junction protein occludin and inhibits VEGF depletion of occludin**

HRMECs cells were treated with IL-18 and expression of tight junction protein occludin was examined (Figure 18). IL-18 increased expression of this tight junction protein whereas by comparison both VEGF and IL-1a reduced expression of occludin. IL-18 can rescue VEGF inhibition of occluding stabilising occluding expression, whereas IL-1a does not rescue VEGF mediated occludin inhibition. These data indicate that IL-18 can stabilise the junctions between endothelial cells enhancing barrier integrity.

The above data indicate that IL-18 reduces oedema by (1) reducing available VEGF and the ability of endothelial cells to respond to it, and (2) driving endothelial cell migration and stabilizing cell-cell junctions upon contact through upregulation of occludin, improving vessel stability and inhibiting permeability of vessel junctions.

## Example 20

### IL-18 induces Fibroblast-like cells to migrate to close a wound created in human Retinal Pigment Epithelium monolayer in vitro

A reduction in the rate of vascular permeability might be due to the retinal pigment epithelium (RPE) encapsulating the neovascular lesions and blocking the leakage. To assess whether IL-18 might affect this process, a series of scratch assays were carried out in cultures of hfRPE cells (Figure 19). While some pigmented RPE were observed to migrate into the wound, the most striking observation was the appearance of fibroblast-like cells that migrated across the wound in response to IL-18 treatment. As the hfRPE cells are a primary culture isolated from primary tissue it appears that contaminating fibroblast-like cells remained in culture through the isolation process.

## Example 21

### IL-18 activates human ocular choroidal fibroblasts.

Given the observation of fibroblast-like cells in the hfRPE scratch assays, the ability of IL-18 to activate human ocular choroidal fibroblasts (HOCFs) was assessed. Figure 20 shows that IL-18 activates HOCFs causing them to change morphology taking on a more stellate active phenotype with intermediate filament vimentin shifting from a perinuclear position to spread deeper towards the cell membrane. IL-18 also strongly reduces the baseline secretion of VEGF from HOCFs. LDH assays demonstrates that IL-18 does not cause HOCF death and may enhance HOCF viability.

## Example 22

### IL-18 activates the mesenchymal contraction phase of wound healing process.

Mesenchymal contraction is an important phase in the wound healing process involving the action of cytoskeletal filaments vimentin and smooth muscle actin (SMA). Mesenchymal stem cells (MSCs) were isolated from mouse bone marrow and treated with IL-18 to assess the effect of IL-18 on these filaments (Figure 21). IL-18 had a strong effect on the appearance of vimentin and SMA in MSCs indicating that IL-18 can drive the mesenchymal contraction phase of wound healing. IL-18 also increased collagen in MSCs indicating that IL-18 can enhance the production of extracellular matrix for cells to migrate along.

Together the above *in vitro* data indicate that IL-18 can enhance the mesenchymal cell migration and mesenchymal cell contraction phases of wound healing which will accelerate the pace of healing resulting in a smaller lesion and smaller scar.

## Example 23

### Increased mesenchymal cell infiltration/activation in animals injected with IL-18

The in vitro findings were verified and it was observed whether IL-18 could enhance the migration of mesenchymal cell infiltration and activation in vivo. Wildtype mice were injected with IL-18 intraperitoneally (Figure 22) and underwent laser-induced injury to generate a choroidal neovascular lesion (CNV). After 3 days the eyes were enucleated, prepared for flatmount and stained for F4/80 to assess infiltrating mononuclear cells and for vimentin to assess infiltrating mesenchymal cells. It was clear that IL-18 increased vimentin and that these vimentin positive cells appeared to form a cage at the periphery of the lesion that encapsulated the endothelial cells (isolectin+ve) and mononuclear cells. This experiment was repeated, this time injecting IL-18 directly into the eye via intravitreal injection on the same day that the mice underwent laser induced injury (Figure 23). A similar pattern of vimentin staining was observed as had been observed with systemic injection, with IL-18 driving vimentin positive cells to encapsulate the neovascular lesion.

## Example 24

### IL-18 reduces the area of fibrovascular scarring

Vimentin is secreted into the extracellular matrix where it contributes to the initial scar tissue. The vimentin staining pattern was assessed 14 days post laser injury and found less vimentin positive staining in WT mice treated with IL-18 compared with vehicle control (Figure 24). Conversely in IL-18 deficient mice (IL-18 KO) the area of vimentin staining is larger compared with vimentin staining in WT mice (Figure 25).

Together these in vitro and in vivo data indicate that IL-18 reduces neovascularisation and oedema by (1) enhancing compensatory proliferation of multiple cell types including endothelial cells fibroblasts macrophages and mesenchymal stem/stromal cells and by (2) accelerating the rate of mesenchymal activation and contraction leading to an accelerated healing of the neovascular lesions, reduced oedema and a smaller scar area. VEGF inhibition alone prevents vascular leakage, but blocks endothelial cell proliferation and maturation, preventing the healing process and keeping the neovascular lesion in a pathological nascent immature state, i.e. a lesion that can't heal. IL-18 is useful as an adjunct therapy to anti-VEGF therapy as it will (1) reduce the expression of VEGFR2 thereby inhibiting any residual VEGF from signalling (2) drive VEGF-independent compensatory endothelial cell proliferation in parallel with enhancing cell-cell junctions upon contact thereby stabilising the vascular lesion, resulting in a smaller stable neovascular membrane, and (3) accelerate the rate of mesenchymal activation and contraction leading to the rapid healing of the neovascular lesions, reduced oedema and a smaller scar area. Therefore the combination of anti-VEGF and IL-18 together will be therapeutic.

Anti-VEGF treatments will remove VEGF from the retina, but will not instigate a healing response. These data indicate that IL-36 is exceptionally well suited to reduce permeability of neovascular lesions, whereas IL-18 is well suited to enhance mesenchymal activation and contraction. A combination of anti-VEGF agent (of any type) treatment with IL-18 and IL-36 would potently inhibit neovascular lesion permeability and promote a wound healing response.

It is well established that vascular barrier breakdown and excessive plasma leakage contributes to a wide variety of disease processes, and is especially critical in impacting neurological disorders and retinal disease. Regulation of vascular permeability and neovascularization can occur via a variety of both direct and indirect mechanisms and various members of the IL-1 family of interleukins are able to mediate aspects of these processes. In fact, proteome profiling has revealed that there are considerable overlaps in pathways and biological functions regulated by IL-1$\beta$ and VEGF in activated endothelial cells. Although VEGF is both a driver of permeability and a pro-angiogenic agent, it is thought to be the regulation of vascular permeability that represents the most immediate and potent effects of anti-VEGF treatment as a therapy. One of the more established areas of IL-36 research concerns its role in psoriasis, where there is evidence that IL-36 may contribute to the sustained vascularisation of psoriatic plaques. Here the aim was to assess whether IL-36 can regulate neovascularization and/or vessel permeability in microvessels associated with the CNS.

Notably, IL-36 provided a striking enhancement of endothelial barrier function. This protection from vessel permeability was observed not only in areas of injury-induced neovascularization but also in the microvessels of the iBRB distal to laser injury. Immune dysregulation and inflammatory processes have been linked with neovascularisation both clinically and experimentally and the vessel permeability observed in the inner retinal vessels is likely a result of laser-induced local inflammation. The neovascularization and nascent vessel permeability observed in response to laser injury is highly dependent on the upregulation of VEGF. Therefore, these data indicate that IL-36$\beta$ has potential to enhance vessel barrier integrity and protect against both VEGF- and inflammation-induced vessel permeability. This is in direct contrast to IL-1's established role in driving vascular permeability, and is also uncommon. Although barrier breakdown is associated with a host of retinal and neurological disorders extremely few barrier-enhancing agents have been discovered to specifically increase barrier integrity and make vessels resistant to leakage. Angiopoietin 1 (Ang1) and a TGF$\beta$R inhibitor (RepSox) are two such factors, both enhance barrier integrity through induced expression of tight junction proteins, and both are undergoing intense study for their therapeutic potential. Similar to the mechanism underlying RepSox and Ang1, these data indicate that IL-36 cytokines also enhance barrier function due, at least in part, to IL-36-dependent transcriptional regulation of tight junction proteins and VE-cadherin, which are directly responsible for maintaining vascular integrity. When protein translation was inhibited by cycloheximide, IL-36 treatment lost its ability to enhance barrier function.

IL-36 appears to have further similarity with Ang1. Although many molecules are known to regulate either angiogenesis or permeability, the overlap between molecules that can regulate both is quite selective. Both VEGF and the angiopoietins are potent pro-angiogenic factors functioning together during early stages of vascular development with VEGF acting early during vessel formation, and Ang1 acting later during vessel remodelling, maturation and stabilization. However, whereas VEGF causes increased vascular permeability, Ang1 stabilizes blood vessels and protects them from VEGF-induced plasma leakage. Furthermore, despite long-term expression of Ang1 resulting in greater number of large blood vessels, acute administration of Ang1 in vivo does not increase neovascular lesion size when administered to adult mice in multiple disease models, instead Ang1 induces highly ordered, hierarchical stable and leakage resistant vascular structures. In this study, it was similarly found that long-term unregulated IL-36 signalling modelled using the il36rn-/- mice, demonstrated a role for IL-36 in vascular modelling. It was also found that IL-36 is a proangiogenic factor in vitro driving primary endothelial cell proliferation, migration, and tube formation. However, despite these apparent pro-angiogenic features of IL-36, it is notable that the introduction of recombinant IL-36$\beta$ into adult mice did not increase neovascular lesion volume in vivo in a pathological setting (mouse model of laser-induced CNV) as might be expected for a pro-angiogenic cytokine.

The biggest difference between normal vessels and new pathological blood vessels is that the new blood vessels lack tight junction proteins, allowing the plasma and serous fluid in the neovascular lesion to leak into the surrounding tissue. IL-36 appears to upregulate angiogenic processes that result in vessel remodelling, and maturation in addition to facilitating the tightening of endothelial cell junctions resulting in stabilised vascular network that does not incite oedema. These data demonstrating this are supported by network analysis on RNA sequencing data. In this context, there is scope to consider IL-36 cytokines as wound healing molecules, which in the context of the retina may instigate regulated vascular healing. Together, these data present IL-36 as a cytokine with barrier enhancing properties that is useful for reducing microvascular leakage in diseases in which the leakage results from inflammation or elevated VEGF.

This evidence indicates that IL-36 is highly expressed on primary human retinal endothelial cells (HRECs) and acts to decrease levels of VEGFR2 and not VEGFR1, which would inhibit the pathological effects of VEGF signalling. Furthermore, IL-36 appears to rescue VEGF-induced permeability of HRECs cultured on Transwell filters, preventing the migration of Evans-blue-BSA across a polarised HREC monolayer. Additionally, IL-18, which is expressed throughout the tissues in the eye, has potent wound healing properties and can induce rapid wound closure while increasing the integrity of tight junction components in endothelial cells of the retina and choroid.

The IL-36 cytokines were first identified following a DNA database search for homologs of IL-1 in 1999. Since then, it has been established that the IL-36 sub-family has three different but highly homologous signalling molecules; IL36α, IL36β and IL36γ. To date, the IL-36 signalling cascade has been studied most extensively in psoriasis, where IL-36R activation drives inflammation in keratinocytes in both the plaque and pustular phenotypes of the disease. Early studies suggest that, at least in psoriasis, IL-36R signalling is potentially acting indirectly as a pro-angiogenic mediator via macrophage activation, however, its role in the regulation of vascular permeability is unknown.

In this study, the mouse retinal vasculature was used as an accessible in vivo model, amenable to manipulation for studying the direct effects of IL-36β cytokine on vessel neovascularisation and permeability in the CNS of adult mice. This model has the advantage of enabling the intravital acquisition of fluorescein angiography images, to accurately gauge vascular permeability in real time at sites of injury and in non-injured vessels. In direct contrast to IL-1 we find that IL-36β acts as a potent inhibitor of vascular permeability, upregulating translation of vital components of the adherens and tight junctions.

Of note, IL-36 is capable of directly promoting angiogenic steps involved in wound healing processes in vitro, however importantly, it has been demonstrated that IL-36R signalling has potential therapeutic application for reducing microvascular leakage in vivo, as the proangiogenic features promoted by IL-36 in vitro are uncoupled from its ability to enhance vascular integrity and reduce vessel permeability in vivo in an acute setting. Furthermore the introduction of recombinant IL-36β by intravitreal injection is well tolerated and non-toxic to surrounding neural tissue. These data expand the functions of IL-36 cytokines to include the uncommon actions of enhancing vessel remodelling and endothelial barrier integrity.

**Claims**

1. A composition comprising IL-36 for use in the treatment or prophylaxis of ocular disorders.

2. A composition for use according to claim 1, wherein the composition further comprises IL-18.

3. A composition for use according to claim 1 or 2, wherein the use comprises administering the composition to a subject in need thereof.

4. A composition for use according to claim 3, wherein the use further comprises administering an anti-VEGF agent to the subject in need thereof.

5. A composition for use according to claim 4, wherein the anti-VEGF agent and the composition are administered simultaneously.

6. A composition for use according to claim 4, wherein the anti-VEGF agent and the composition are administered sequentially.

7. A composition for use according to any of claims 1-6, wherein the use is use in wound healing.

8. A composition for use according to any of claims 1-7, wherein the use comprises administration of a cell expressing IL-36 and/or IL-18.

9. A composition for use according to any of claims 1-8, wherein the ocular disorders are selected from any one or more of age-related macular degeneration (AMD), nascent geographic atrophy, incomplete retinal pigment epithelium and outer retinal atrophy (iRORA), wet AMD, geographic atrophy, dry AMD, macular oedema, retinopathy, diabetic retinopathy, and glaucoma.

10. A composition for use according to any of claims 4-9, wherein the anti-VEGF agent is selected from an anti-VEGF antibody or fragment thereof, an anti-VEGF polyclonal antibody or fragment thereof, an anti-VEGF monoclonal antibody or fragment thereof, a VEGF inhibitor, a small molecule VEGF inhibitor, a tyrosine kinase inhibitor, a small molecule tyrosine kinase inhibitor, fusion proteins comprising VEGF or fragment thereof, recombinant fusion proteins comprising VEGF or fragment thereof, and combinations each thereof.

11. A composition for use according to any of claims 4-10, wherein the anti-VEGF agent is selected from aflibercept, axitinib, bevacizumab, cabozantinib, lapatinib, lenvatinib, pazopanib, ponatinib, ramucirumab, ranibizumab, regorafenib, sorafenib, sunitinib, vandetanib, and combinations each thereof.

12. A composition for use according to any of claims 1-11, wherein the use comprises administration by a route selected from topical, parenteral, intra-arterial, intravenous, intraocular, intravitreal, and combinations each thereof.

13. A composition for use according to any of claims 1-12, wherein the use comprises administration at a unit dose of IL-36 and/or IL-18 of at least 0.3 ng.

14. A composition for use according to any of claims 1-12, wherein the IL-36 is selected from IL36$\alpha$, IL36$\beta$ or IL36$\gamma$.

**Patentansprüche**

1. Zusammensetzung umfassend IL-36 zur Verwendung in der Behandlung oder Prophylaxe von Augenerkrankungen.

2. Zusammensetzung zur Verwendung nach Anspruch 1, wobei die Zusammensetzung ferner IL-18 umfasst.

3. Zusammensetzung zur Verwendung nach Anspruch 1 oder 2, wobei die Verwendung das Verabreichen der Zusammensetzung an ein bedürftiges Subjekt umfasst.

4. Zusammensetzung zur Verwendung nach Anspruch 3, wobei die Verwendung ferner das Verabreichen eines Anti-VEGF-Wirkstoffs an das bedürftige Subjekt umfasst.

5. Zusammensetzung zur Verwendung nach Anspruch 4, wobei der Anti-VEGF-Wirkstoff und die Zusammensetzung gleichzeitig verabreicht werden.

6. Zusammensetzung zur Verwendung nach Anspruch 4, wobei der Anti-VEGF-Wirkstoff und die Zusammensetzung sequenziell verabreicht werden.

7. Zusammensetzung zur Verwendung nach einem der Ansprüche 1-6, wobei die Verwendung eine Verwendung in der Wundheilung ist.

8. Zusammensetzung zur Verwendung nach einem der Ansprüche 1-7, wobei die Verwendung das Verabreichen einer IL-36- und/oder IL-18-exprimierenden Zelle umfasst.

9. Zusammensetzung zur Verwendung nach einem der Ansprüche 1-8, wobei die Augenerkrankungen ausgewählt sind aus altersabhängiger Makuladegeneration (AMD), früher geografischer Atrophie, unvollständiger Atrophie des retinalen Pigmentepithels und der äußeren Retina (iRORA), feuchter AMD, geografischer Atrophie, trockener AMD, Makulaödem, Retinopathie, diabetischer Retinopathie und Glaukom.

10. Zusammensetzung zur Verwendung nach einem der Ansprüche 4-9, wobei der Anti-VEGF-Wirkstoff ausgewählt ist aus einem Anti-VEGF-Antikörper oder einem Fragment davon, einem Anti-VEGFpolyklonalen Antikörper oder einem Fragment davon, einem Anti-VEGF-monoklonalen Antikörper oder einem Fragment davon, einem VEGF-Inhibitor, einem niedermolekularen VEGF-Inhibitor, einem Tyrosinkinase-Inhibitor, einem niedermolekularen Tyrosinkinase-Inhibitor, Fusionsproteinen umfassend VEGF oder ein Fragment davon, rekombinanten Fusionsproteinen umfassend VEGF oder ein Fragment davon, sowie Kombinationen jeder davon.

11. Zusammensetzung zur Verwendung nach einem der Ansprüche 4-10, wobei der Anti-VEGF-Wirkstoff ausgewählt ist aus Aflibercept, Axitinib, Bevacizumab, Cabozantinib, Lapatinib, Lenvatinib, Pazopanib, Ponatinib, Ramucirumab, Ranibizumab, Regorafenib, Sorafenib, Sunitinib, Vandetanib sowie Kombinationen jeder davon.

12. Zusammensetzung zur Verwendung nach einem der Ansprüche 1-11, wobei die Verwendung ein Verabreichen über eine ausgewählte Applikationsroute umfasst, ausgewählt aus topischer, parenteraler, intraarterieller, intravenöser, intraokularer, intravitrealer Route sowie Kombinationen jeder davon.

13. Zusammensetzung zur Verwendung nach einem der Ansprüche 1-12, wobei die Verwendung ein Verabreichen mit einer Einzeldosis von mindestens 0,3 ng IL-36 und/oder IL-18 umfasst.

14. Zusammensetzung zur Verwendung nach einem der Ansprüche 1-12, wobei das IL-36 ausgewählt ist aus IL-36$\alpha$, IL-36$\beta$ oder IL-36$\gamma$.

**Revendications**

1. Composition comprenant IL-36 destinée à être utilisée dans le traitement ou la prophylaxie des troubles oculaires.

2. Composition destinée à être utilisée selon la revendication 1, dans laquelle la composition comprend également IL-18.

3. Composition destinée à être utilisée selon la revendication 1 ou 2, dans laquelle l'utilisation consiste à administrer la composition à un sujet qui en a besoin.

4. Composition destinée à être utilisée selon la revendication 3, dans laquelle l'utilisation comprend également l'administration d'un agent anti-VEGF à un sujet qui en a besoin.

5. Composition destinée à être utilisée selon la revendication 4, dans laquelle l'agent anti-VEGF et la composition sont administrés simultanément.

6. Composition destinée à être utilisée selon la revendication 4, dans laquelle l'agent anti-VEGF et la composition sont administrés séquentiellement.

7. Composition destinée à être utilisée selon l'une quelconque des revendications 1 à 6, dans laquelle l'utilisation est une utilisation dans la cicatrisation des plaies.

8. Composition destinée à être utilisée selon l'une quelconque des revendications 1 à 7, dans laquelle l'utilisation comprend l'administration d'une cellule exprimant IL-36 et/ou IL-18.

9. Composition destinée à être utilisée selon l'une quelconque des revendications 1 à 8, dans laquelle les troubles oculaires sont sélectionnés parmi un ou plusieurs des troubles suivants : dégénérescence maculaire liée à l'âge (AMD), atrophie géographique naissante, atrophie incomplète de l'épithélium pigmentaire rétinien et de la rétine externe (iRORA), AMD humide, atrophie géographique, AMD sèche, œdème maculaire, rétinopathie, rétinopathie diabétique et glaucome.

10. Composition destinée à être utilisée selon l'une quelconque des revendications 4 à 9, dans laquelle l'agent anti-VEGF est choisi parmi un anticorps anti-VEGF ou un fragment de celui-ci, un anticorps polyclonal anti-VEGF ou un fragment de celui-ci, un anticorps monoclonal anti-VEGF ou un fragment de celui-ci, un inhibiteur de VEGF, un inhibiteur de VEGF à petite molécule, un inhibiteur de tyrosine kinase, un inhibiteur de tyrosine kinase à petite molécule, des protéines de fusion comprenant du VEGF ou un fragment de celui-ci, des protéines de fusion recombinantes comprenant du VEGF ou un fragment de celui-ci, et des combinaisons de chacun de ces éléments.

11. Composition destinée à être utilisée selon l'une quelconque des revendications 4 à 10, dans laquelle l'agent anti-VEGF est choisi parmi l'aflibercept, l'axitinib, le bevacizumab, le cabozantinib, le lapatinib, le lenvatinib, le pazopanib, le ponatinib, le ramucirumab, le ranibizumab, le regorafénib, le sorafénib, le sunitinib, le vandétanib et les combinaisons de chacun d'eux.

12. Composition destinée à être utilisée selon l'une quelconque des revendications 1 à 11, dans laquelle l'utilisation comprend l'administration par une voie choisie parmi les voies topique, parentérale, intra-artérielle, intraveineuse, intraoculaire, intravitréenne et les combinaisons de chacune d'elles.

13. Composition destinée à être utilisée selon l'une quelconque des revendications 1 à 12, dans laquelle l'utilisation comprend l'administration d'une dose unitaire d'IL-36 et/ou d'IL-18 d'au moins 0,3 ng.

14. Composition destinée à être utilisée selon l'une quelconque des revendications 1 à 12, dans laquelle l'IL-36 est choisie parmi l'IL36$\alpha$, l'IL36$\beta$ ou l'IL36$\gamma$.

Figure 1

Figure 1

Figure 2

Figure 2

Figure 3

Figure 3

Figure 3

Figure 4

**Figure 4**

Figure 5

Figure 5

Figure 6

Figure 6

Figure 7

Figure 7

Figure 8

# HRMEC IL-36R expression with VEGF treatment

Figure 9

## VEGFR2 expression with IL-36α treatment

## VEGFR2 expression with IL-36β treatment

Figure 10

Figure 11

**IL-18 (BCVA)**

**IL-18 (OCT)**

**IL-18 (Ob. Impression)**

Figure 12

| Correlation of ΔOCT and IL-18 at BL, W6 and W12. | | | | | | |
|---|---|---|---|---|---|---|
| | BL | | W6 | | W12 | |
| | Spearmann r | (p Value) | Spearmann r | (p Value) | Spearmann r | (p Value) |
| IL-18 | 0.29 | (0.37) | -0.40 | (0.21) | -0.25 | (0.26) |

A.

IL18
r = 0.2961

B.

IL18
r = -0.4091

C.

IL18
r = -0.2533

Figure 13

**A.**

**BCVA**

**B.**

**OCT**

**C.**

**ΔBCVA & Free IL-18**

r = 0.1111

**D.**

**ΔOCT & Free IL-18**

r = -0.4112     P = 0.01744 (*)

Figure 14

**A.**

### 3 wk old JR5558 mice

ug IL-18 per kg of body weight
IP injection

**B.**

**C.**

Figure 15

VEGFR2

**Figure 15**
**CONTINUATION**

Figure 16

Figure 16

Figure 17

Figure 18

Figure 18
CONTINUATION

Figure 19

**A.**

8 h post treatment

untreated    10 ng/ml IL-18    100 ng/ml IL-18    1000 ng/ml IL-18

Figure 20

Figure 20

**Figure 20**

**D.**

Figure 21

Saline

IL-18 (100 µg/kg)

3 day CNV

Figure 22

Figure 23

3 day CNV

IL-18 (0.3 ng)

Vehicle/ control

Vehicle

IL-18 (0.3 ng)

14 day CNV

Figure 24

67

Figure 25

IL-18 Knockout mice

WT mice

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TORTOLA L** ; **ROSENWALD E** ; **ABEL B** ; **BLUMBERG H** ; **SCHÄFER M** ; **COYLE AJ** ; **RENAULD JC** ; **WERNER S** ; **KISIELOW J** ; **KOPF M**. Psoriasiform dermatitis is driven by IL-36-mediated DC-keratinocyte crosstalk.. *J Clin Invest*, 2012, vol. 122, 3965-3976
- **ABBOTT, N. J.** ; **HUGHES, C. C.** ; **REVEST, P. A.** ; **GREENWOOD, J.** Development and characterisation of a rat brain capillary endothelial culture: towards an in vitro blood-brain barrier.. *J Cell Sci,*, 1992, vol. 103 (1), 23-37

- **ASSMANN, J. C.** ; **MULLER, K.** ; **WENZEL, J.** ; **WALTHER, T.** ; **BRANDS, J.** ; **THORNTON, P.** ; **SCHWANINGER, M.** Isolation and Cultivation of Primary Brain Endothelial Cells from Adult Mice.. *Bio Protoc*, 2017, vol. 7 (10)
- **TSAI, C. E.** ; **DAOOD, M. J.** ; **LANE, R. H.** ; **HANSEN, T. W.** ; **GRUETZMACHER, E. M.** ; **WATCHKO, J. F.** P-glycoprotein expression in mouse brain increases with maturation.. *Biol Neonate,*, 2002, vol. 81 (1), 58-64